# EUROPÄISCHE PATENTANMELDUNG

(11) **EP 3 424 906 A1**
(43) Veröffentlichungstag der Anmeldung: **09.01.2019**
(21) Anmeldenummer: 17179568.5
(22) Anmeldetag: 04.07.2017
(51) Int. Cl.: C07D 215/44, C07D 215/46, C07D 401/12, C07D 413/12, A61K 31/47, A61K 31/4709, A61P 31/06

(54) **MYCOBACTERIUM-TUBERCOLOSIS-THIOREDOXINREDUKTASE-INHIBITOR ALS ANTITUBERKULOTIKUM**

(71) Anmelder: Technische Universität Dortmund, 44227 Dortmund (DE)
(72) Erfinder: KOCH, Oliver, 44141 Dortmund (DE); BERING, Luis, 44225 Dortmund (DE)
(74) Vertreter: Kutzenberger Wolff & Partner

(57) **Zusammenfassung**

Die Erfindung betrifft *Mycobacterium-tubercolosis*-Thioredoxinreduktase-Inhibitoren, Verfahren zu deren Herstellung, Arzneimittel enthaltend diese Verbindungen und die Verwendung dieser Verbindungen zur Herstellung von Arzneimitteln.

## Beschreibung

Die Erfindung betrifft *Mycobacterium-tubercolosis-*Thioredoxinreduktase-Inhibitoren, Verfahren zu deren Herstellung, Arzneimittel enthaltend diese Verbindungen und die Verwendung dieser Verbindungen zur Herstellung von Arzneimitteln.

Tuberkulose ist eine bakterielle Erkrankung, an der jährlich ca. 1,5 Millionen Menschen sterben, vor allem Menschen in den Entwicklungsländern und Menschen mit einem geschwächten Immunsystem (z.B. HIV-Patienten). Aktuell in der Therapie eingesetzte Antituberkulotika sind zum größten Teil schon über 50 Jahre alt. In Anbetracht der notwendigen 6 Monate dauernden Therapie ist die Resistenzentwicklung absehbar. Erste extrem resistente Stämme sind bereits in den Industrieländern aufgetreten. Die Erreger der Tuberkulose, *Mycobacterium tuberculosis* und andere Arten von Mycobakterien, werden von den Makrophagen des Immunsystems zwar erkannt und aufgenommen (phagozytiert), sie werden aber aufgrund der besonderen Beschaffenheit ihrer Zellwand in den Makrophagen nicht verdaut, sondern können in den Makrophagen überleben. Die Behandlung der Tuberkulose ist sehr aufwändig und langwierig, und die Ausbildung von Antibiotika-resistenten Stämmen wird, wie bei anderen bakteriellen Infektionen, in Zukunft zu einem wichtigen Problem werden. Die besondere Herausforderung in der Entwicklung neuer Antibiotika gegen die Tuberkulose liegt darin, dass diese Antibiotika zwar in die Makrophagen eindringen und die Bakterien abtöten müssen, jedoch nicht die Makrophagen selbst schädigen dürfen.

*Mycobacterium-tubercolosis-*Thioredoxinreduktase *(MtTrxR)* und *Mycobacterium-tubercolosis*-Thioredoxinreduktase-Inhibitoren sind im Stand der Technik bekannt.
Lin et al., (2016), "Mycobacterium tubercolosis Thioredoxin Reductase Is Essential for Thiol Redox Homeostasis but Plays a Minor Role in Antioxidant Defense", PloS Pathog 12 (6), offenbart verschiedene Aspekte der *Mycobacterium-tubercolosis-*Thioredoxinreduktase.
Ferner offenbart Koch, O., et al. (2013), "Identification of M. tuberculosis thioredoxin reductase inhibitors based on high-troughput docking using constraints", J. Med. Chem. 56: 4849-59, 2013, die Thioredoxinreduktase als potentielles Target für die Entwicklung neuer Anti-Tuberkulotika. Als vielversprechendste Verbindung bzw. als vielversprechendster *Mycobacterium-tubercolosis-*Thioredoxinreduktase-Inhibitor wurde darin das Aminochinolin **1** mit einem ICₛₒ-Wert von 12,5 µM (nach T. Jäger, H. Budde, L. Flohe, U. Menge, M. Singh, M. Trujillo, R. Radi, Arch. Biochem. Biophys. 2004, 423, 182-191) gefunden.
Im Zusammenhang mit Thioredoxinreduktase-Inhibitoren wird des Weiteren auf Gustafsson TN, Osman H, Werngren J, Hoffner S, Engman L, Holmgren A., "Ebselen and analogs as inhibitors of Bacillus anthracis thioredoxin reductase and bactericidal antibacterials targeting Bacillus species, Staphylococcus aureus and Mycobacterium tuberculosis", Biochim Biophys Acta. 2016 Jun; 1860(6):1265-71, verwiesen.

Die bekannten Verbindungen sind jedoch nicht in jeglicher Hinsicht zufrieden stellend und es besteht ein Bedarf an weiteren Verbindungen mit vergleichbaren oder besseren Eigenschaften. Diese Aufgabe wird durch den Gegenstand der Patentansprüche gelöst.

Es wurde überraschend gefunden, dass die erfindungsgemäßen Verbindungen eine klare Struktur-Wirkungsbeziehung auf die Wachstumshemmung von *Mycobacterium tuberculosis* in infizierten humanen Makrophagen zeigen, ohne eine erkennbare schädliche Wirkung auf die Makrophagen aufzuweisen. Ferner deuten erste Erkenntnisse darauf hin, dass die erfindungsgemäßen Verbindungen auch gegen resistente Stämme und Bakterien wie *Staphylococcus aureus* wirksam sind.
Abbildung 1 ist auf die antimykobakterielle Aktivität der erfindungsgemäßen Verbindungen **2, 8** und **68** gerichtet.
Abbildung 2 ist auf die antimykobakterielle Aktivität der erfindungsgemäßen Verbindung **68** gegenüber dem *Mycobacterium-tubercolosis-*Strang H37Rv in infizierten humanen Makrophagen gerichtet.
Abbildung 3 ist auf die cytotoxische Aktivität der erfindungsgemäßen Verbindung **68** gegenüber humanen Makrophagen gerichtet.

Die Erfindung betrifft in einem ersten Aspekt Verbindungen der allgemeinen Formel (1), 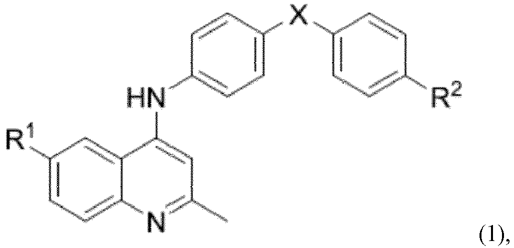 worin
R₁ und R₂ jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NHC(=O)N(R₀)₂; X für -CH₂-, -S(=O)₂- oder -C(=O)- steht;
wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist; "Cycloaliphat" jeweils ein gesättigter oder ein- oder mehrfach ungesättigter, unsubstituierter oder ein-oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf);
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR_{0,} -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀, -NH-C(=O)N(R₀)₂, -Si(R₀)₃ und -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃ oder -PO(OR₀)₂ verstanden wird; wobei ggf. vorhandene N-Ringatome jeweils oxidiert sein können;
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
oder ein physiologisch verträgliches Salz davon.

Unter "jeweils unabhängig" im Zusammenhang mit R₀ ist zu verstehen, dass eine Einschränkung der Definition von R₀ im Rahmen einer bevorzugten Ausführungsform und bezüglich eines bestimmten Rests nicht bedeutet, dass die Definition von R₀ bezüglich irgendeines anderen Rests (analog) eingeschränkt wird, außer es wird ausdrücklich daraufhingewiesen, dass dem so ist. Ferner ist bei jeder Bezugnahme auf "Aliphat", "Cycloaliphat", "Aryl" und "Heteroaryl" im Rahmen einer bevorzugten Ausführungsform zu verstehen, dass diese unsubstituiert oder ein- oder mehrfach substituiert sind, außer es wird ausdrücklich darauf hingewiesen, dass dem nicht so ist. Des Weiteren umfasst die Definition von "ein- oder mehrfach substituiert" im Zusammenhang mit dem Generischen (z.B. "-Aryl") auch die entsprechende Substitution des Spezifischen (z.B. "-Phenyl"), außer es wird ausdrücklich darauf hingewiesen, dass dem nicht so ist.

Die erfindungsgemäßen Verbindungen zeigen gute inhibitorische Aktivität gegenüber *MtTrxR.* In einer bevorzugten Ausführungsform weisen die erfindungsgemäßen Verbindungen einen IC₅₀-Wert nach *Lu et al.* von 0,10 bis 10,00 µM auf. Methoden zur Bestimmung des IC₅₀-Werts nach *Lu et al.* sind dem Fachmann bekannt. Diesbezüglich wird auf J. Lu, A. Vlamis-Gardikas, K. Kandasamy, R. Zhao, T. N. Gustafsson, L. Engstrand, S. Hoffner, L. Engman, A. Holmgren, FASEB J. 2013, 27, 1394-1403, verwiesen.

Bevorzugt sind R¹ und R² jeweils unabhängig voneinander ausgewählt aus der Gruppe bestehend aus -H, -F, -Cl, -Br, -I, -R₀, -C(=O)R₀, -C(=O)OR₀, -C(=O)NHR₀, -C(=O)N(R₀)₂, -OR₀, -OC(=O)R₀, -OC(=O)OR₀, -OC(=O)NHR₀, -OC(=O)N(R₀)₂, -SR₀, -S(=O)₁₋₂-R₀, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NH-C(=O)N(R₀)₂; wobei R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl oder -C₁₋₈-Aliphat-Heteroaryl steht.

In einer weiteren bevorzugten Ausführungsform steht X für -CH₂- oder -S(=O)₂-; R¹ ist ausgewählt aus der Gruppe bestehend aus -R₀, -C(=O)R₀, -C(=O)OR₀, -C(=O)NHR₀, -C(=O)N(R₀)₂, -OR₀, -OC(=O)R₀, -OC(=O)OR₀, -OC(=O)NHR₀ und -OC(=O)N(R₀)₂; wobei R₀ jeweils unabhängig für -C₁₋₈-Aliphat, -Aryl, -Heteroaryl, -C₁₋₈-Aliphat-Aryl oder -C₁₋₈-Aliphat-Heteroaryl steht; und R² ist ausgewählt aus der Gruppe bestehend aus -NH₂, -NHR₀, -N(R₀)₂, -N⁺(R₀)₃ und -N⁺(R₀)₂O⁻; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -C₃₋₁₂-Cycloaliphat, -Aryl, -Heteroaryl oder -C₁₋₈-Aliphat-C₃₋₁₂-Cycloaliphat steht; oder X steht für -C(=O)-; R¹ ist ausgewählt aus der Gruppe bestehend aus -R₀, -C(=O)R₀, -C(=O)OR₀, -C(=O)NHR₀, -C(=O)N(R₀)₂, -OR₀, -OC(=O)R₀, -OC(=O)OR₀, -OC(=O)NHR₀ und -OC(=O)N(R₀)₂; wobei R₀ jeweils unabhängig für -C₁₋₈-Aliphat, -Aryl, -Heteroaryl, -C₁₋₈-Aliphat-Aryl oder -C₁₋₈-Aliphat-Heteroaryl steht; und R² ist ausgewählt aus der Gruppe bestehend aus -NH₂, -NHR₀, -N(R₀)₂, -N⁺(R₀)₃ und -N⁺(R₀)₂O⁻; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl, -Oxadiazolyl, -C₃₋₁₂-Cycloaliphat, -Aryl oder -C₁₋₈-Aliphat-C₃₋₁₂-Cycloaliphat steht.

In einer weiteren bevorzugten Ausführungsform steht X für -CH₂- oder -S(=O)₂-; R¹ ist ausgewählt aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und R² ist ausgewählt aus der Gruppe bestehend aus -NH₂, -NHR₀, -N(R₀)₂, -N⁺(R₀)₃ und -N⁺(R₀)₂O⁻; wobei Rojeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -Aryl oder-Heteroaryl steht; oder X steht für -C(=O)-; R¹ ist ausgewählt aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und R² ist ausgewählt aus der Gruppe bestehend aus -NH₂, -NHR₀, -N(R₀)₂, -N⁺(R₀)₃ und -N⁺(R₀)₂O-; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht.

In einer weiteren bevorzugten Ausführungsform steht X für -CH₂- oder -S(=O)₂-; R¹ ist ausgewählt aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und R² ist ausgewählt aus der Gruppe bestehend aus -NH₂, -NHR₀, -N(R₀)₂, -N⁺(R₀)₃ und -N⁺(R₀)₂O⁻; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -Phenyl, -Naphthyl, -Anthracenyl, -Phenanthrenyl, -Fluoranthenyl, -Fluorenyl, -Indanyl, -Tetralinyl, -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, Pyrimidinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht; oder X steht für -C(=O)-; R¹ ist ausgewählt aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂-CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und R² ist ausgewählt aus der Gruppe bestehend aus -NH₂, -NHR₀, -N(R₀)₂, -N⁺(R₀)₃ und -N⁺(R₀)₂O⁻; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht.

In einer weiteren bevorzugten Ausführungsform steht X für -CH₂- oder -S(=O)₂-; R¹ ist ausgewählt aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und R² ist ausgewählt aus der Gruppe bestehend aus -NH₂, -NHR₀, -N(R₀)₂, -N⁺(R₀)₃ und -N⁺(R₀)₂O⁻; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -Phenyl, -Naphthyl, -Anthracenyl, -Phenanthrenyl, -Fluoranthenyl, -Fluorenyl, -Indanyl, -Tetralinyl, -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, Pyrimidinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht; oder X steht für -C(=O)-; R¹ ist ausgewählt aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂-CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und R² ist ausgewählt aus der Gruppe bestehend aus -NH₂, -NHR₀, -N(R₀)₂, -N⁺(R₀)₃ und -N⁺(R₀)₂O⁻; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht; und -Phenyl, -Naphthyl, -Anthracenyl, -Phenanthrenyl, -Fluoranthenyl, -Fluorenyl, -Indanyl, -Tetralinyl, -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrimidinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl und -Oxadiazolyl jeweils unabhängig voneinander unsubstituiert oder einfach substituiert sind durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀ und -OR₀ verstanden wird, wobei ggf. vorhandene N-Ringatome jeweils oxidiert sein können; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht.

In einer weiteren bevorzugten Ausführungsform steht X für -CH₂- oder -S(=O)₂-; oder für -CH₂-oder -C(=O)-; oder für -S(=O)₂- oder -C(=O)-; oder für -CH₂-; oder für -S(=O)₂-; oder für -C(=O)-.

### Bevorzugte Verbindungen sind

6-Methoxy-2-methyl-N-(4-((4-(pyridin-4-ylamino)phenyl)sulfonyl)phenyl) chinolin-4-amin (2) 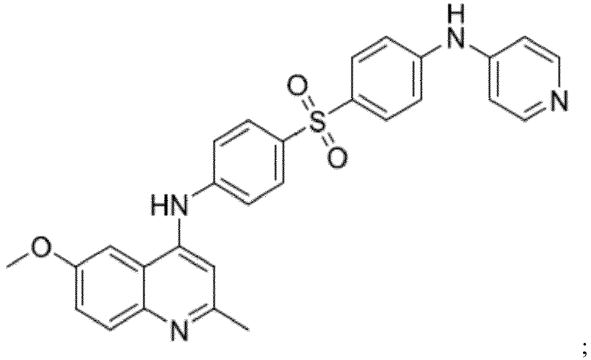 und/oder
N-(4-(4-Aminobenzyl)phenyl)-6-methoxy-2-methylchinolin-4-amin (8) 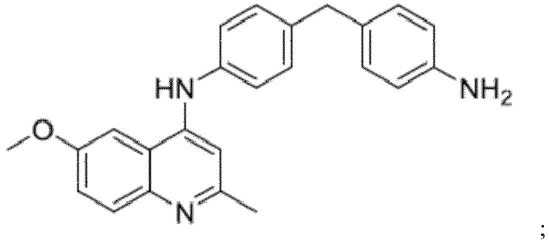 und/oder
N-(4-((4-Aminophenyl)sulfonyl)phenyl)-6-methoxy-2-methylchinolin-4-amin (**9**) 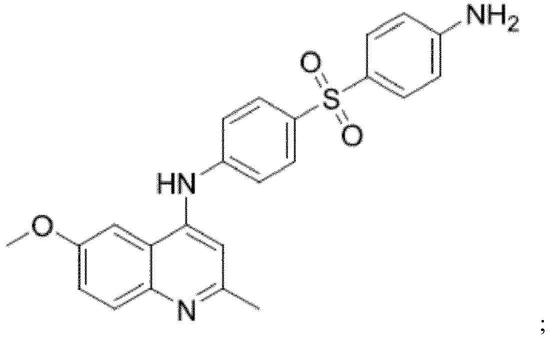 und/oder
N-(4-((4-((6-Methoxy-2-methylchinolin-4-yl)amin)phenyl)sulfonyl)phenyl)acetamid (**10**) 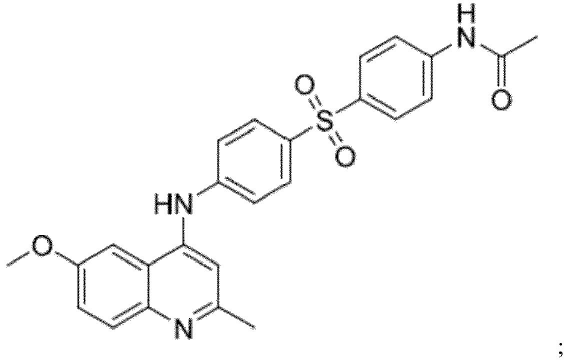 und/oder
4-Butyl-N-(4-((4-((6-methoxy-2-methylchinolin-4-yl)amin)phenyl)sulfonyl)phenyl)benzamid (**11**) 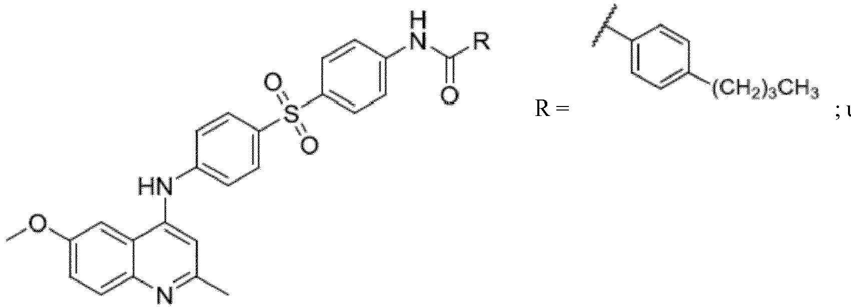 und/oder
1-Benzyl-3-(tert-butyl)-N-(4-((4-((6-methoxy-2-methylchinolin-4-yl)amin)phenyl) sulfonyl)phenyl)-1H-pyrazol-5-carboxamid (**12**) 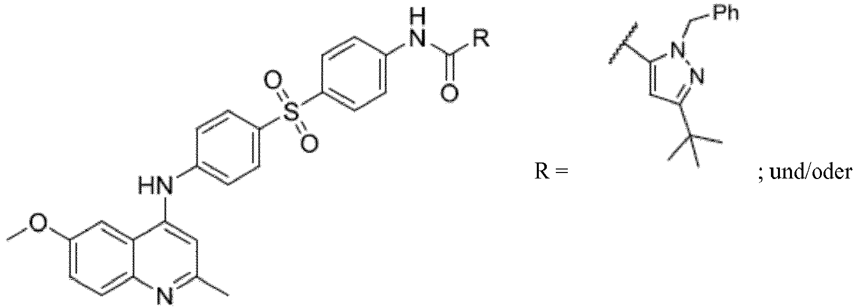
6-Methoxy-2-methyl-N-(4-((4-(pyrimidin-4-yl-amin)phenyl)sulfonyl)phenyl) chinolin-4-amin (**24**) 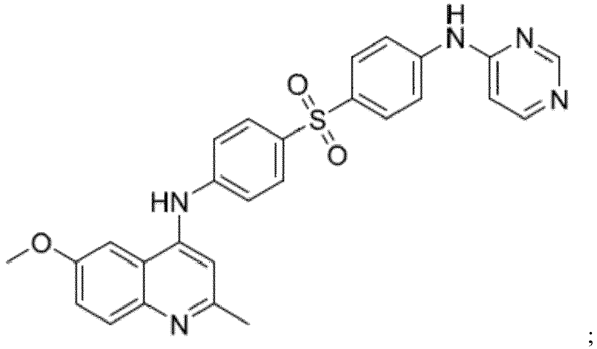 und/oder
N-(4-((4-((3-(tert-Butyl)-1H-pyrazol-5-yl)amino)phenyl)sulfonyl)phenyl)-6methoxy-2-methylchinolin-4-amin (**25**) 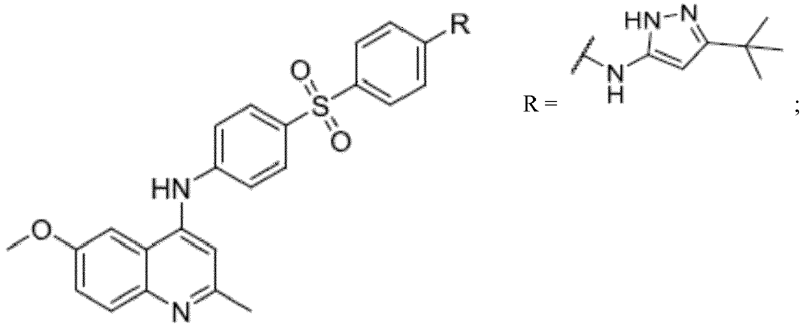 und/oder
6-Methoxy-N-(4-((4-((4-methoxyphenyl)amin)phenyl)sulfonyl)phenyl)-2-methylchinolin-4-amin (**26**) 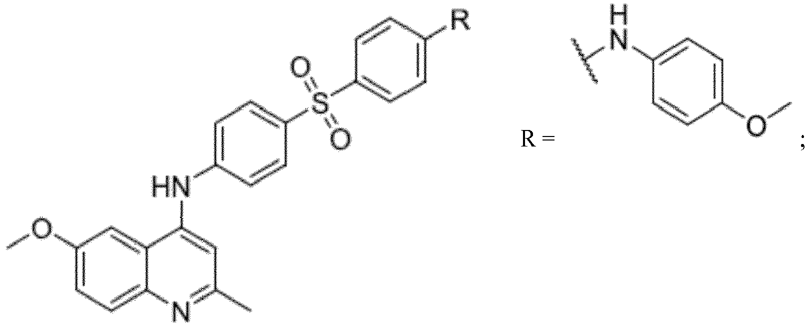 und/oder
Methyl-4-((4-((4-((6-methoxy-2-methylchinolin-4-yl)amino)phenyl)sulfonyl)phenyl)-amino)benzoat (**27**) 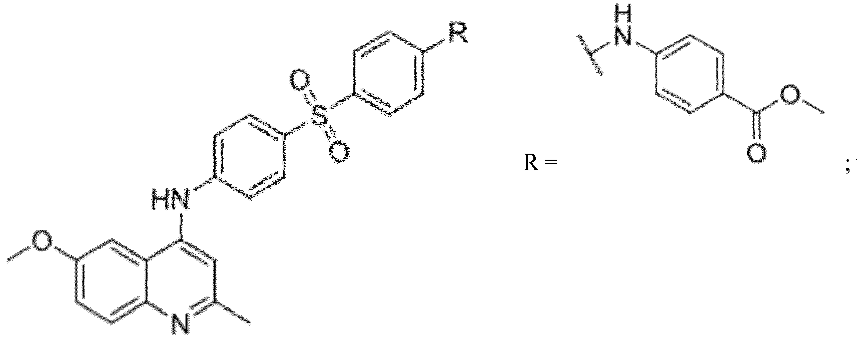 und/oder
N-(4-((4-((2-Methyl-6-(piperidin-1-yl)chinolin-4-yl)amino)phenyl)sulfonyl) phenyl)acetamid (**36**) 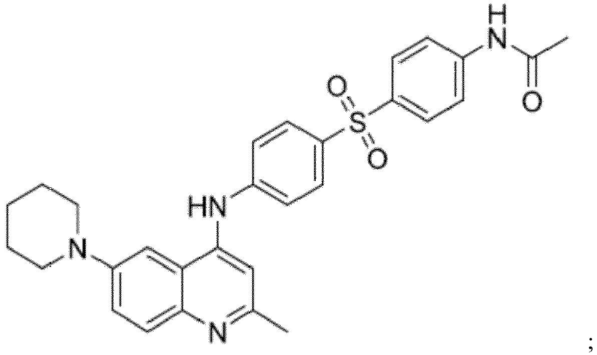 und/oder
6-Brom-2-methyl-N-(4-((4-(pyridin-4-ylamin)phenyl)sulfonyl)phenyl)chinolin-4amin (**37**) 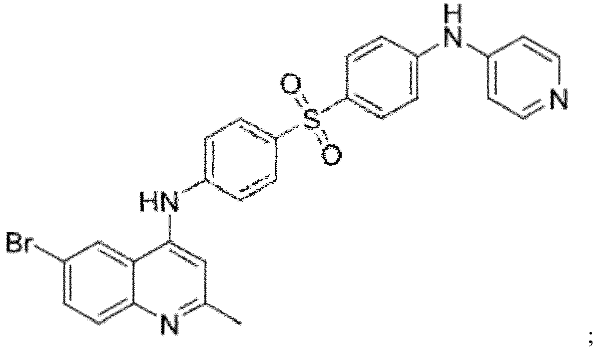 und/oder
N-(4-((4-((2-Methyl-6-morpholinchinolin-4-yl)amino)phenyl)sulfonyl)phenyl)acetamid (**40**) 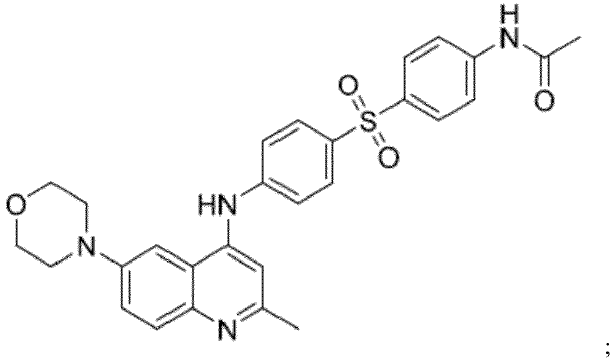 und/oder
(4-((6-Methoxy-2-methylchinolin-4-yl)amin)phenyl)(4-(pyridin-4-yl-amino)phenyl)methanon (**45**) 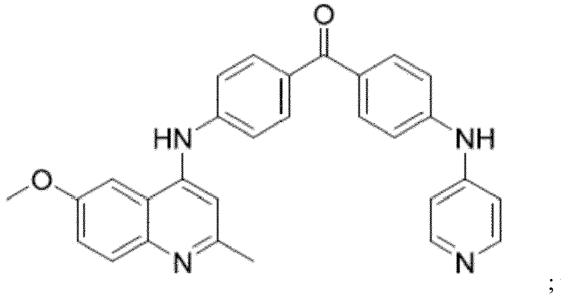 und/oder
(4-((6-Methoxy-2-methylchinolin-4-yl)amino)phenyl)(4-(pyrimidin-4-yl-amino) phenyl)methanon (**46**) 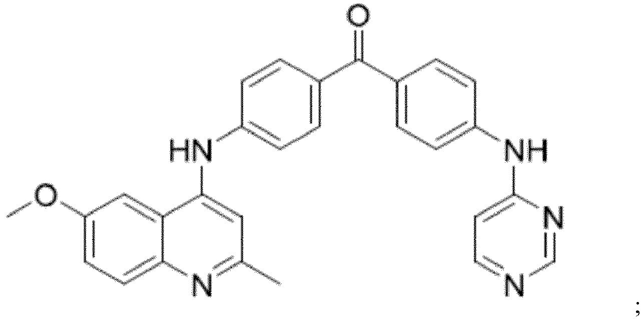 und/oder
6-Methoxy-2-methyl-N-(4-(4-(pyridin-4-ylamino)benzyl)phenyl)chinolin-4-amin (**63**) 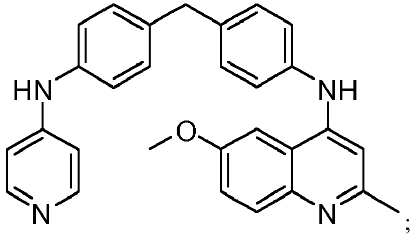 und/oder
6-methoxy-2-methyl-N-(4-(4-(pyrimidin-4-ylamino)benzyl)phenyl)quinolin-4-amine (**68**) 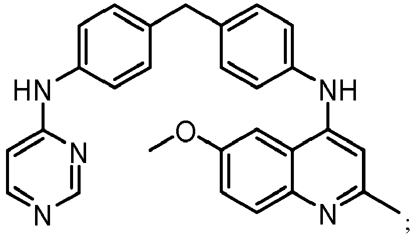 und/oder
deren physiologisch verträgliche Salze.

### Besonders bevorzugte Verbindungen sind

6-Methoxy-2-methyl-N-(4-((4-(pyridin-4-ylamino)phenyl)sulfonyl)phenyl) chinolin-4-amin (**2**); und/oder
N-(4-(4-Aminobenzyl)phenyl)-6-methoxy-2-methylchinolin-4-amin (8); und/oder
6-Methoxy-2-methyl-N-(4-(4-(pyridin-4-ylamino)benzyl)phenyl)chinolin-4-amin (**63**); und/oder
6-methoxy-2-methyl-N-(4-(4-(pyrimidin-4-ylamino)benzyl)phenyl)quinolin-4-amine (**68**); und/oder deren physiologisch verträgliche Salze.

Insbesondere bevorzugte Verbindungen sind 6-methoxy-2-methyl-N-(4-(4-(pyrimidin-4-ylamino)benzyl)phenyl)quinolin-4-amine (**68**) und/oder dessen physiologisch verträgliche Salze.

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl).

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nichtaromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist).

Innerhalb der cyclischen Substituenten gilt demnach bevorzugt folgende Priorität bei der Zuordnung: Heteroaryl > Aryl > Cycloaliphat.

Zum Zwecke der Beschreibung werden einbindige und mehrbindige, z.B. zweibindige Kohlenwasserstoffreste nicht begrifflich unterschieden, d.h. "C₁₋₃-Aliphat" umfasst, je nach Sinnzusammenhang, z.B. sowohl -C₁₋₃-Alkyl, -C₁₋₃-Alkenyl und -C₁₋₃-Alkinyl, als auch z.B. -C₁₋₃-Alkylen-, -C₁₋₃-Alkenylen- und C₁₋₃-Alkinylen-.

Bevorzugt ist Aliphat jeweils ein verzweigter oder unverzweigter, gesättigter oder ein ein- oder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend aus -F, -Cl, -Br, -I, -CN, -NO₂, -CHO, =O, -R₀, -C(=O)R₀, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=C))OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂. So umfasst "Aliphat" acyclische gesättigte oder ungesättigte Kohlenwasserstoffreste, die verzweigt oder geradkettig sein können, d.h. Alkanyle, Alkenyle und Alkinyle. Dabei weisen Alkenyle mindestens eine C=C-Doppelbindung und Alkinyle mindestens eine C≡C-Dreifachbindung auf. Bevorzugte unsubstituierte einbindige Aliphaten umfassen -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂-CH₂CH₃ und -CH₂CH₂-CH₂CH₂CH₂CH₃; aber auch -CH=CH₂, -C=CH, -CH₂CH=CH₂, -CH=CH-CH₃, -CH₂C≡CH, -C≡CCH₃ und -CH=CHCH=CH₃. Bevorzugte unsubstituierte zweibindige Aliphaten umfassen -CH₂-, -CH₂CH₂-, -CH₂CH(CH₃)-, -CH(CH₃)-CH₂-, -CH₂CH₂CH₂-, -CH(CH₃)CH₂CH₂-, -CH₂CH(CH₃)-CH₂-, -CH₂CH₂CH(CH₃)-, -CH-(CH₂CH₃)CH₂- und -CH₂CH₂-CH₂CH₂-; aber auch -CH=CH-, -C≡C-, -CH₂CH=CH-, -CH=CHCH₂-, -CH₂C≡C- und -C≡C-CH₂-. Bevorzugte substituierte einbindige Aliphaten umfassen -CH₂F, -CHF₂, -CF₃, -CH₂CF₃, -CF₂CF₃, -CH₂OH, -CH₂CH₂OH, -CH₂CHOHCH₃, -CH₂OCH₃ und CH₂CH₂OCH₃. Bevorzugte substituierte zweibindige Aliphaten umfassen -CF₂-, -CF₂CF₂-, -CH₂CHOH-, -CHOHCH₂-und -CH₂CHOHCH₂-. Besonders bevorzugt sind Methyl, Ethyl, n-Propyl und n-Butyl.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono-oder multicyclischer Kohlenwasserstoffrest. Die Zahl der Ringkohlenstoffatome liegt vorzugsweise im angegebenen Bereich (d.h. ein "C₃₋₈-"Cycloaliphat weist vorzugsweise 3, 4, 5, 6, 7 oder 8 Ringkohlenstoffatome auf). Zum Zwecke der Beschreibung ist "C₃₋₈-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂. Vorteilhaft ist C₃₋₈-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I, -OH, -OC₁₋₆-Alkyl, -OC(=O)C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH verstanden. Bevorzugt sind Verbindungen, wobei "Aliphat substituiert" oder "Cycloaliphat substituiert" Aliphat oder Cycloaliphat substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von -CF₃ oder -CH₂CF₃, oder an verschiedenen Stellen, wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfasst -OAliphat u.a. auch -OCH₂CH₂O-CH₂CH₂-OH. Bevorzugt ist es, wenn Aliphat oder Cycloaliphat substituiert sind mit -F, -Cl, -Br, -I, -CN, -CH₃, -C₂H₅, -NH₂, -NO₂, -SH, -CF₃, -OH, -OCH₃, -OC₂H₅ oder -N(CH₃)₂. Ganz besonders bevorzugt ist es, wenn Aliphat oder Cycloaliphat substituiert sind mit -OH, -OCH₃ oder -OC₂H₅.

Bevorzugt steht Aryl jeweils unabhängig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein-oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I, -CN, -NO₂, -CHO, =O, -Ro, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)NHR₀, -OC(=O)-N(R₀)₂, -SH, -SRo, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NH-C(=O)R₀, -NHC(=O)OR₀, -NHC(=O)NH₂, -NHC(=O)NHR₀, -NH-C(=O)N(R₀)₂, -Si(R₀)₃, -PO(OR₀)₂.

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die Substituenten von Aryl und Heteroaryl jeweils unabhängig voneinander ausgewählt aus -F, -Cl, -Br, -I, -CN, -CHO, -CO₂H, -NH₂, -NO₂, -NHR₀, -N(R₀)₂, -N⁺(R₀)₃, -N⁺(R₀)₂O⁻, -SH, -SRo, -OH, -OR₀, -C(=O)R₀, -CO₂R₀, -C(=O)NH₂, -C(=O)NHR₀, -C(=O)N(R₀)₂, -S(=O)₁₋₂R₀, -S(=O)₂NH₂, -SO₃H, =O oder -Ro. Bevorzugte Substituenten sind -F, -Cl, -Br, -I, -OH, -OC₁₋₆-Alkyl, -O-C(=O)-C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH. Bevorzugt sind Verbindungen, wobei "Aryl substituiert" oder "Heteroaryl substituiert" Aryl oder Heteroaryl substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Die erfindungsgemäßen Verbindungen können je nach Substitutionsmuster chiral oder achiral sein.

Bei den erfindungsgemäßen Verbindungen kann es sich je nach Substitution um Isomere handeln, bei denen das Substitutionsmuster mit syn/anti bezeichnet werden kann. "Syn/anti-Isomere" sind eine Untergruppe der Stereoisomere (Konfigurationsisomere).

In einer bevorzugten Ausführungsform beträgt der Diastereomerenüberschuss des syn-Isomers wenigstens 50 %de, bevorzugter wenigstens 75 %de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des anti-Isomers wenigstens 50 %de, bevorzugter wenigstens 75 %de, noch bevorzugter wenigstens 90%de, am bevorzugtesten wenigstens 95%de und insbesondere wenigstens 99%de.

Geeignete Methoden zur Trennung der Isomere (Diastereomere) sind dem Fachmann bekannt. Als Beispiele können Säulenchromatographie, präparative HPLC und Kristallisationsverfahren genannt werden.

Sind die erfindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-)Natrium-, (Mono-) oder (Di-)Kalium-, Magnesium- oder Calcium-Salze.

Die Erfindung betrifft in einem zweiten Aspekt Arzneimittel enthaltend wenigstens eine erfindungsgemäße Verbindung oder ein physiologisch verträgliches Salz davon, sowie ggf. geeignete Zusatz- und/oder Hilfsstoffe und/oder ggf. weitere Wirkstoffe. Alle bevorzugten Ausführungsformen, welche vorstehend im Zusammenhang mit der erfindungsgemäßen Verbindung beschrieben wurden, gelten entsprechend analog auch für das erfindungsgemäße Arzneimittel.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe, Bindemittel und/oder Antioxidantien. Als Bindemittel eignen sich beispielsweise Sirup Akazia, Gelatine, Sorbit, Tragant und Polyvinylpyrrolidon. Als Füllstoffe eignen sich beispielsweise Lactose, Zucker, Maisstärke, Calciumphosphat, Sorbit und Glycin. Als Antioxidantien eignen sich beispielsweise Ascorbinsäure, Butylhydroxyanisol (BHA), Butylhydroxytoluol (BHT), Salze der Ascorbinsäure, Monothioglycerin, phosphorige Säure, Vitamin C, Vitamin E und dessen Derivate, Natriumbisulfit, besonders bevorzugt Butylhydroxytoluol oder Butylhydroxyanisol und α-Tocopherol. Die geeigneten Zusatz- und/oder Hilfsstoffe werden vorzugsweise in Mengen von 0,01 bis 10 Gew.-%, vorzugsweise 0,03 bis 5 Gew.-%, bezogen auf das Gesamtgewicht des Arzneimittels, eingesetzt.

Die erfindungsgemäßen Arzneimittel können als flüssige Arzneiformen in Form von Injektionslösungen, Tropfen oder Säften, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Antibiotikum, vorzugsweise ein Antibiotikum ausgewählt aus der Gruppe bestehend aus Pyrazinamid, Isoniazid, Ethambutol, Rifampicin, Aminoglykosiden und Fluorochinolonen, enthält.

In einer bevorzugten Form des Arzneimittels liegt eine enthaltene erfindungsgemäße Verbindung als reines Diastereomer und/oder Enantiomer vor.

Die Erfindung betrifft in einem dritten Aspekt die erfindungsgemäße Verbindung und/oder das erfindungsgemäße Arzneimittel zur Anwendung als Medikament.

Die Erfindung betrifft in einem vierten Aspekt die erfindungsgemäße Verbindung und/oder das erfindungsgemäße Arzneimittel zur Anwendung bei der Behandlung von Tuberkolose; bevorzugt Tuberkolose ausgewählt aus der Gruppe bestehend aus Tuberkulose der Atmungsorgane, Tuberkulose des Nervensystems, Tuberkulose sonstiger Organe, und Miliartuberkulose; bevorzugt Tuberkulose der Atmungsorgane ausgewählt aus der Gruppe bestehend aus Lungentuberkulose, Tuberkulose der intrathorakalen Lymphknoten, Tuberkulose des Larynx, der Trachea und der Bronchien, und Tuberkulöser Pleuritis; und/oder Tuberkulose des Nervensystems ausgewählt aus der Gruppe bestehend aus Tuberkulöser Meningitis, und Meningealem Tuberkulom; und/oder Tuberkulose sonstiger Organe ausgewählt aus der Gruppe bestehend aus Tuberkulose der Knochen und Gelenke, Tuberkulose des Urogenitalsystems, Tuberkulose peripherer Lymphknoten, Tuberkulose des Darmes, des Peritoneums und der Mesenteriallymphknoten, Tuberkulose der Haut und des Unterhautgewebes, Tuberkulose des Auges, Tuberkulose des Ohres, und Tuberkulose der Nebennieren.

Die Erfindung betrifft in einem fünften Aspekt ein Verfahren zur Herstellung der erfindungsgemäßen Verbindungen wie in der folgenden Beschreibung und Beispielen ausgeführt.

### Präparative Methoden

Dünnschichtchromatographie (DC): Es wurden Aluminium-DC-Folien (Kieselgel 60, Fluoreszenzindentifikator F264 (60F-254)) der Firma Merck eingesetzt. Die Verbindungen wurden durch UV-Bestrahlung bei 254 und 360 nm detektiert.

Flüssigchromatographie Massenspektrometrie (LC-MS): Zur Aufnahme niedrig aufgelöster Massenspektren wurde eine Shimadzu LCMS-8030 verwendet (Säule: Luna C18(2), 100A, Länge: 250 mm, Innendurchmesser: 4,6 mm, Partikelgröße: 2,5-15 µm, Ionisierungsmethode: Elektrospray-Ionisierung).

Hochauflösende Massenspektrometrie (HR-MS): Zur Aufnahme der hoch aufgelösten Massenspektren wurde ein LTQ Orbititrap verwendet, der an ein Accela HPLC-System (Säule: Hypersil Gold, Länge: 50 mm, Innendurchmesser: 1 mm, Partikelgröße: 1,9 µm, Ionisierungsmethode: Elektrospray-Ionisierung) gekoppelt war. Der analysierte Wellenlängenbereich lag im Bereich von 200-600 nm. Die Massen wurden im m/z-Bereich von 1502000 Th detektiert.

Kernspinresonanzspektroskopie (NMR): Zur Charakterisierung der synthetisierten Verbindungen mit ¹H- und ¹³C-NMR wurde ein Varian Mercury 400 (400 MHz), ein Bruker Avance DRX 500 (500 MHz) und ein Bruker 600 (600 MHz) Spektrometer eingesetzt. Als deuterierte Lösungsmittel kamen CDCl₃ und DMSO-d₆ zum Einsatz, deren detektierte Restprotonen als interne Referenz dienten. Die chemische Verschiebung δ wurde in ppm angegeben und die Kopplungskonstante J in Hz. Die Multiplizität, die bei auftretender SpinSpin-Kopplung zu sehen war, wurde mit folgenden Abkürzungen gekennzeichnet: s (Singulett), d (Dublett), t (Triplett), q (Quartett), dd (Doublet vom Doublet), m (Multiplett).

Säulenchromatographie: Für die stationäre Phase der Säulenchromatographie wurde Kieselgel der Firma VWR mit einer Partikelgröße von 40-63 µm eingesetzt.

### Reagenzien

Alle verwendeten Lösungsmittel wurden, wie vom Händler erhältlich, eingesetzt. Sofern nicht anders angegeben, wurden sonstig verwendete Reagenzien und Lösungsmittel ohne zusätzliche Aufreinigung verwendet.

### Allgemeine Arbeitsvorschriften

Methode A: Aminierung von 4-Chlor-2-methyl-chinolinen. Zu einer Lösung von 4-Chlor-2-methylchinolin (7) und Amin in absolutem EtOH, wurde ein Tropfen konzentrierte HCl zugegeben und die Reaktionsmischung über Nacht unter Rückfluss erhitzt. Die Reaktion wurde unter vermindertem Druck konzentriert und der verbliebene Rückstand mit DCM und NH₄OH suspendiert und gerührt, bis sämtlicher Feststoff gelöst war. Die wässrige Phase wurde dreimal mit DCM extrahiert, die vereinigten organischen Phasen über MgSO₄ getrocknet und am Vakuum konzentriert. Säulenchromatographie an Kieselgel (DCM/MeOH) lieferte das gewünschte Produkt.

Methode B: Synthese und Amidkupplung von Carbonsäurechloriden. Zu einer Lösung von Carbonsäure (2 Äquiv.) und katalytische Mengen DMF in DCM, wurde tropfenweise Oxalylchlorid (2 Äquiv.) zugegeben und die Reaktion für 15 h bei RT gerührt. Das erhaltene Carbonsäurechlorid wurde bei 0 °C tropfenweise zu einer Mischung aus N-(4-((4Aminophenyl)sulfonyl)phenyl)-6-methoxy-2-methyl-chinolin-4-amin (**9**) (1 Äquiv.) und katalytische Mengen DMAP in Pyridin gegeben. Die Reaktionsmischung wurde langsam auf RT erwärmt und weitere 4 h gerührt. Anschließend wurde die Reaktionsmischung unter vermindertem Druck konzentriert und der verbliebene Rückstand in DCM und gesättigter NaHCO₃-Lösung suspendiert. Die wässrige Phase wurde dreimal mit DCM extrahiert, die vereinigten organischen Phasen über MgSO₄ getrocknet und unter vermindertem Druck konzentriert. Säulenchromatographie an Kieselgel (DCM/MeOH) lieferte das gewünschte Produkt.

Methode C: Buchwald-Hartwig-Kreuzkupplungen von Arylhalogenen und Aminen. In einem ausgeheizten Schlenkkolben mit Rührfisch wurde Arylhalogen (1. Äquiv.), Amin (1,2 Äquiv.), Base und Pd-Katalysator (0,1 Äquiv.) und Ligand (0,1 - 0,3 Äquiv.) eingewogen und mit einem Septum verschlossen. Der Kolben wurde dreimal mit Argon evakuiert, entgastes 1,4-Dioxan zugegeben und das Septum gegen einen Glasstopfen getauscht. Die Reaktionsmischung wurde danach bei 100 °C für 24 h gerührt. Nach Abkühlen auf RT wurde die Reaktion mit DCM verdünnt, über Celite^{©} gefiltert und mit gesättigter NaHCO₃-Lösung aufgenommen. Die wässrige Phase wurde dreimal mit DCM extrahiert, die vereinigten organischen Phasen über MgSO₄ getrocknet und das Lösungsmittel unter vermindertem Druck entfernt. Säulenchromatographie an Kieselgel (DCM/MeOH) lieferte das gewünschte Produkt.

### Synthese der erfindungsgemäßen Verbindungen und der Vergleichsverbindung

4-((6-Methoxy-2-methylchinolin-4-yl)amino)phenol (**1**): 4-Chlor-6-methoxy-2methylchinolin (**7**) (50 mg, 0,24 mmol, 1 Äquiv.) und p-Aminophenol (39 mg, 0,36 mmol, 1,5 Äquiv.) wurden in absolutem EtOH (4 mL) gelöst und entsprechend Methode A umgesetzt, wodurch das Produkt als gelber Feststoff erhalten wurde (53 mg, 0,19 mmol, 80%). R_{f} = 0,2 (DCM/MeOH, 40:1). LC-MS: m/z = 281,38 [M+H]⁺. HR-MS [M+H]⁺ = kalkuliert 281,12845, gefunden 281,12919 (+2,61325 ppm). ¹H NMR (DMSO-d₆, 500 MHz) δ ppm 9.40 (s, 1H), 8.42 (s, 1H), 7.68 (d, J = 2.7 Hz, 1H), 7.65 (d, J = 9.1 Hz, 1H), 7.25 (dd, J= 9.1, 2.7 Hz, 1H), 7.13 (d, J = 8.7 Hz, 2H), 6.85 (d, J = 8.7 Hz, 2H), 6.42 (s, 1H), 5.75 (s, 1H), 3.89 (s, 3H), 2.34 (s, 3H). ¹³C NMR (DMSO-d₆, 126 MHz) δ ppm 155.82, 155.61, 154.53, 148.63, 144.05, 131.37, 129.77, 126.12, 120.47, 118.08, 115.93, 100.87, 100.02, 55.56, 24.80.

6-Methoxy-2-methyl-N-(4-((4-(pyridin-4-ylamino)phenyl)sulfonyl)phenyl) chinolin-4-amin (**2**): N-(4-((4-Bromphenyl)sulfonyl)phenyl)-6-methoxy-2-methylchinolin-4amin (**22**) (20 mg, 0,041 mmol, 1 Äquiv.), 4-Aminopyridin (5 mg, 0,05 mmol, 1,2 Äquiv.), NaOtBu (5,5 mg, 0,057 mmol, 1,4 Äquiv.), Pd₂(dba)₃ (4 mg, 0,004 mmol, 0,1 Äquiv.) und Triphenylphosphan (2,2 mg, 0,008 mmol, 0,3 Äquiv.) wurden entsprechend Methode C in 1,4-Dioxan (1,5 mL) umgesetzt, wodurch das Produkt als gelblicher Feststoff erhalten wurde (10 mg, 0,019 mmol, 48%). R_{f} = 0,22 (DCM/MeOH, 10:1). LC-MS: m/z = 249,15 [M+H]⁺. HR-MS [M+H]⁺ = berechnet 497,16419, gefunden 497,16442 (+0,46338 ppm). ¹H NMR (DMSO-d₆, 500 MHz)) δ ppm 9.35 (s, 1 H), 9.26 (s, 1 H), 8.29 (dd, J = 8.8, 6.3 Hz, 2 H), 7.85 (dd, J = 8.8, 6.3 Hz, 2 H), 7.72 - 7.78 (m, 2 H), 7.55 (d, J = 2.7 Hz, 1 H), 7.52 (d, J = 8.8 Hz, 1 H), 7.44 (d, J = 8.8 Hz, 1 H), 7.32 - 7.36 (m, 2 H), 7.29 (d, J = 8.8 Hz, 2 H), 7.02 - 7.08 (m, 2 H), 6.62 (d, J = 8.8 Hz, 1 H), 6.09 (s, 1 H), 3.88 (s, 3 H), 2.49 (s, 3H). ¹³C NMR (DMSO-d₆, 126 MHz) δ ppm 156.2, 153.3, 150.3, 150.3, 148.3, 148.1, 145.5, 144.8, 135.0, 133.4, 133.2, 129.0, 128.9, 128.8, 128.3, 126.4, 52.6, 20.7.

6-Methoxy-2-methylchinolin-4(1H)-on (**6**): Zu einer Suspension von 4-p-Anisidine (6 g, 40 mmol, 1 Äquiv.) und Ethylacetoacetat (6,21 mL, 48 mmol, 1,2 Äquiv.) in Toluol (75 mL) wurde konzentrierte AcOH (3 mL) zugeben. Unter azeotroper Entfernung von Wasser (Dean-Stark Apparatur) wurde die Reaktionsmischung 20 h unter Rückfluss erhitzt. Anschließend wurde das Lösungsmittel unter vermindertem Druck entfernt und der erhaltene Feststoff in Ph₂O (15 mL) suspendiert und für 1 h auf 240 °C erhitzt. Die Reaktionsmischung wurde auf 50 °C abgekühlt und warmes Hexan (50 mL) zugegeben. Der entstandene Niederschlag wurde ab filtriert und mit viel Hexan und Aceton gewaschen, wodurch das Produkt als weißer Feststoff (3,5 g, 18 mmol, 46%) erhalten wurde. ¹H NMR (DMSO-d₆, 500 MHz) δ ppm 11.53 (s, 1H), 7.55 - 7.37 (m, 2H), 7.24 (dd, J = 9.0, 2.9 Hz, 1H), 5.87 (s, 1H), 3.81 (s, 3H), 2.32 (s, 3H). ¹³C NMR (DMSO-d₆, 126 MHz) δ ppm 176.96, 156.08, 149.41, 135.57, 126.35, 122.52, 120.29, 108.26, 105.21, 56.14, 20.20.

4-Chlor-6-methoxy-2-methylchinolin (7): 6-Methoxy-2-methylchinolin-4(1H)-on (**6**) (3 g, 14 mmol, 1 Äquiv.) wurde bei 0 °C portionsweise zu einer Überschusslösung von POCl₃ (20 mL) geben. Anschließend wurde die Suspension 30 Min bei RT gerührt und danach 1 h unter Rückfluss erhitzt. Der Überschuss an POCl₃ wurde durch Destillation entfernt und der verbliebene Rückstand langsam auf eine Mischung aus Eis und gesättigter NaHCO₃-Lösung gegossen und ausgiebig gerührt. Der pH-Wert wurde mit NH₄OH auf 9 eingestellt und der entstandene Niederschlag abgefiltert wodurch das Produkt als rötlicher Feststoff erhalten wurde (3,2 g, 14 mmol, 86%). R_{f} = 0,43 (PE/EtOAc, 1:1). LC-MS: m/z = 208,6 [M+H]⁺. 1H NMR (DMSO-d₆, 500 MHz) δ ppm 7.87 (d, J = 9.1 Hz, 1H), 7.57 (s, 1H), 7.43 (dd, J = 9.1, 2.8 Hz, 1H), 7.33 (d, J = 2.8 Hz, 1H), 3.91 (s, 3H), 2.59 (s, 3H). ¹³C NMR (DMSO-d₆, 126 MHz) δ ppm 157.67, 156.07, 144.00, 139.46, 130.42, 124.77, 122.61, 122.04, 101.37, 55.51, 24.12.

N-(4-(4-Aminobenzyl)phenyl)-6-methoxy-2-methylchinolin-4-amin (**8**): 4-Chlor-6methoxy-2-methylchinolin (**7**) (50 mg, 0,24 mmol, 1 Äquiv.) und Diamin-diphenylmethan (190 mg, 0,96 mmol, 4 Äquiv.) wurden in absolutem EtOH (3 mL) gelöst und entsprechend Methode A umgesetzt, wodurch das Produkt als goldgelber Feststoff erhalten wurde (83 mg, 0,2 mmol, 85%). R_{f} = 0,3 (DCM/MeOH, 20/1). LC-MS: m/z = 370,19 [M+H]⁺. ¹H NMR (DMSO-d₆, 500 MHz) δ ppm 7.82 (d, J = 9.1 Hz, 2H), 7.40 (dd, J = 9.1, 2.6 Hz, 1H), 7.38 - 7.28 (m, 4H), 7.01 (d, J = 8.3 Hz, 2H), 6.82 (s, 1H), 6.63 (d, J = 8.3 Hz, 2H), 5.83 (s, 1H), 3.99 (s, 3H), 3.86 (s, 2H), 2.01 (s, 3H). ¹³C NMR (DMSO-d₆, 126 MHz) δ ppm 172.02, 155.99, 155.51, 147.89, 146.65, 142.98, 138.12, 138.00, 129.35, 129.14, 128.82, 128.28, 122.91, 121.11, 118.53, 114.09, 113.96, 101.27, 101.09, 55.67, 54.84, 24.14, 21.11.

N-(4-((4-Aminophenyl)sulfonyl)phenyl)-6-methoxy-2-methylchinolin-4-amin (**9**): 4-Chlor-6-methoxy-2-methylchinolin (**7**) (178 mg, 0,86 mmol, 1 Äquiv.) und Diamindiphenylsulfon (640 mg, 2,5 mmol, 3 Äquiv.) wurden in absolutem EtOH (15 mL) gelöst und entsprechend Methode A umgesetzt, wodurch das Produkt als weißer Feststoff erhalten wurde (320 mg, 0,76 mmol, 88%). R_{f} = 0,5 (DCM/MeOH, 10:1). LC-MS m/z = 420,15 [M+H]⁺. HRMS [M+H]⁺ = berechnet 420,13764, gefunden 420,13755 (-0,21456 ppm). ¹H NMR (DMSO-d₆, 500 MHz) δ ppm 8.62 (s, 1H), 7.36 (d, J = 8.8 Hz, 2H), 7.33 (d, J = 9.1 Hz, 1H), 7.13 (d, J = 8.7 Hz, 3H), 6.99 (d, J = 8.8 Hz, 2H), 6.90 (dd, J = 9.1, 2.7 Hz, 1H), 6.71 (s, 1H), 6.22 (d, J = 8.8 Hz, 2H), 5.68 (s, 2H), 3.45 (s, 3H), 2.75 (s, 3H). ¹³C NMR (DMSO-d₆, 126 MHz) d₆) δ ppm 156.14, 156.07, 153.28, 145.97, 144.56, 144.36, 135.07, 129.91, 129.07, 128.25, 126.50, 121.16, 120.01, 118.77, 112.97, 105.99, 101.17, 55.53, 24.60.

N-(4-((4-((6-Methoxy-2-methylchinolin-4-yl)amin)phenyl)sulfonyl)phenyl)acetamid (**10**): N-(4-((4-Aminophenyl)sulfonyl)phenyl)-6-methoxy-2-methylchinolin-4-amin (**9**) (30 mg, 0,071 mmol, 1 Äquiv.) wurde in Pyridin (1 mL) gelöst, Ac₂O (0,28 mmol, 40 µL, 4 Äquiv.) zugegeben und die Reaktion für 18 h bei RT gerührt. Anschließend wurde Pyridin unter vermindertem Druck entfernt und der Rückstand mit gesättigter NaHCO₃-Lösung und DCM aufgenommen. Die wässrige Phase wurde dreimal mit DCM extrahiert, die vereinigten organischen Phasen über MgSO₄ getrocknet und unter vermindertem Druck konzentriert. Säulenchromatographie an Kieselgel (DCM/MeOH) lieferte das gewünschte Produkt als weißen Feststoff (28,5 mg, 0,061 mmol, 87%). R_{f} = 0,3 (DCM/MeOH, 10:1). LC-MS: m/z = 462,48 [M+H]⁺. HR-MS [M+H]⁺ = berechnet 462,14820, gefunden 462,14815 (-0,11724 ppm). ¹H NMR (DMSO-d₆, 500 MHz) δ ppm 10.36 (s, 1H), 9.12 (s, 1H), 7.92 - 7.82 (m, 4H), 7.83 - 7.72 (m, 3H), 7.55 (d, J = 2.8 Hz, 1H), 7.49 - 7.41 (m, 2H), 7.35 (dd, J = 9.1, 2.8 Hz, 1H), 7.19 (s, 1H), 3.89 (s, 3H), 2.50 (s, 3H), 2.09 (s, 3H). ¹³C NMR (DMSO-d₆, 126 MHz) δ ppm 169.01, 156.16, 143.44, B135.43, 132.87, 128.88, 128.23, 121.18, 120.19, 118.91, 118.38, 101.14, 55.50, 24.62, 24.07.

4-Butyl-N-(4-((4-((6-methoxy-2-methylchinolin-4-yl)amin)phenyl)sulfonyl)phenyl)benzamid (**11**): 4-Butyl-benzoesäure (25,5 mg, 0,14 mmol, 2 Äquiv.) wurde in DCM (5 mL) gelöst und entsprechend Methode B mit N-(4-((4-Aminophenyl)sulfonyl)phenyl)-6-methoxy2-methylchinolin-4-amin (**9**) (30 mg, 0,071 mmol, 1 Äquiv.) umgesetzt, wodurch das Produkt als gelber Feststoff erhalten wurde (29 mg, 0,05 mmol, 70%). R_{f} = 0,55 (DCM/MeOH, 10:1). LC-MS: m/z = 580,65 [M+H]⁺. HR-MS [M+H]⁺ = berechnet 580,22645, gefunden 580,22592 (-0,92275 ppm). ¹H NMR (DMSO-d₆, 500 MHz) δ ppm 10.50 (s, 1H), 7.93 (s, 3H), 7.83 (d, J = 9.1 Hz, 3H), 7.59 (s, 1H), 7.46 (d, J = 7.5 Hz, 2H), 7.37 (d, J = 9.1 Hz, 1H), 7.28 (t, J = 7.5 Hz, 3H), 7.25 - 7.14 (m, 2H), 7.14 - 7.00 (m, 3H), 6.73 (s, 1H), 5.68 (s, 1H), 3.89 (s, 3H), 2.09 (s, 3H), 1.30 (d, J = 2.0 Hz, 6H), 1.26 (d, J = 2.0 Hz, 3H). ¹³C NMR (DMSO-d₆, 126 MHz) δ ppm 159.61, 158.33, 156.30, 142.79, 137.98, 136.28, 134.73, 128.95, 128.31, 128.13, 127.19, 127.13, 126.86, 126.68, 121.53, 120.32, 120.04, 118.89, 107.41, 105.35, 101.31, 55.57, 53.73, 53.59, 31.74, 31.65, 30.29, 30.22.

1-Benzyl-3-(tert-butyl)-N-(4-((4-((6-methoxy-2-methylchinolin-4-yl)amin)phenyl) sulfonyl)phenyl)-1H-pyrazol-5-carboxamid (**12**): 1-Benzyl-3-tert-butyl-1H-pyrrazol-5carbonsäure (36 mg, 0,14 mmol, 2 Äquiv.) wurde in DCM (5 mL) gelöst und entsprechend Methode B mit N-(4-((4-Aminophenyl)-sulfonyl)phenyl)-6-methoxy-2-methylchinolin-4amin (**9**) (30 mg, 0,071 mmol, 1 Äquiv.) umgesetzt, wodurch das Produkt als gelber Feststoff erhalten wurde (31 mg, 0,047 mmol, 67%). R_{f} = 0,5 (DCM/MeOH, 10:1). LC-MS: m/z = 660,25 [M+H]⁺. HR-MS [M+H]⁺ = berechnet 660,26390, gefunden 660,26466 (+1,14775 ppm). ¹H NMR (DMSO-d₆, 500 MHz) δ ppm 10.51 (br. s., 1 H), 9.49 (br. s., 1H) 9.29 - 9.67 (m, 1H), 7.94 (br. s., 4 H), 7.58 - 7.84 (m, 2H), 7.33 - 7.58 (m, 2H), 6.92 - 7.34 (m, 7H), 5.68 (br. s., 2H), 3.90 (s., 3H), 2.43 (s, 3H), 1.29 (br. s., 9H). ¹³C NMR (DMSO-d₆, 126 MHz) δ ppm 183.1, 165.2, 155.6, 153.2, 151.0, 150.3, 146.0, 145.6, 142.4, 141.1, 138.0, 136.3, 133.7, 131.8, 130.6, 128.3, 128.2, 126.9, 126.8, 124.1, 120.4, 116.2, 114.8, 114.3, 107.9, 105.4, 101.5, 57.8, 55.7, 51.9, 30.3, 27.4, 24.3.

1-Brom-4-((4-nitrophenyl)sulfonyl)benzol (**18**): 4-Nitrobenzolsulfonylchlorid (3,58 g, 16,2 mmol, 1 Äquiv.) und AlCl₃ (3,23 g, 24,2 mmol, 1,5 Äquiv.) wurden in Brombenzol (4,23 mL, 40,4 mmol, 2,5 Äquiv.) gelöst und 30 Minuten bei RT gerührt. Anschließend wurde die Reaktionsmischung über 1,5 h schrittweise auf 100 °C erhitzt und für weitere 4 h gerührt. Nach Abkühlen auf RT wurde die Reaktion auf Eiswasser gefällt, der entstandene Feststoff abfiltriert und mit Wasser und iPrOH gewaschen. Das Rohprodukt wurde danach in EtOH/EtOAc umkristallisiert, wodurch das Produkt als gelbbrauner Feststoff erhalten wurde (4 g, 11,7 mmol, 72%). R_{f} = 0,87 (PE/EA, 1:1). LC-MS: m/z = 365,35 [M+Na]⁺. ¹H NMR (DMSO-d₆, 500 MHz) δ ppm 8.40 (d, J = 5.0 Hz, 2H), 8.24 (d, J = 5.0 Hz, 2H), 7.95 (d, J = 5.0 Hz, 2H), 7.88 (J = 5.0 Hz, 2H). ¹³C NMR (DMSO-d6, 126 MHz) δ ppm 176.04, 155.15, 148.48, 134.64, 125.43, 121.59, 119.36, 107.33, 104.28.

N-(4-((4-Aminophenyl)sulfonyl)phenyl)pyridin-4-amin (**20**): N-(4-((4-Nitrophenyl)sulfonyl)-phenyl)pyridin-4-amin (64 mg, 0,18 mmol, 1 Äquiv.) wurde in EtOH/H₂O (3:1 mL) gelöst und Na₂S₂O₄ (184 mg, 0,9 mmol, 5 Äquiv.) zugegeben und die Reaktionsmischung für 30 h bei 75 °C gerührt. Nach Abkühlen auf RT wurde die Reaktion unter vermindertem Druck konzentriert, DCM und gesättigte NaHCO₃-Lösung zugegeben und der pH-Wert mit NH₄OH auf 9 eingestellt. Die wässrige Phase wurde dreimal mit DCM extrahiert und die vereinigten organischen Phasen über MgSO₄ getrocknet. Säulenchromatographie an Kieselgel (DCM/MeOH) lieferte das gewünschte Produkt als weißen Feststoff (45 mg, 0,13 mmol, 76%). R_{f}= 0,18 (DCM/MeOH, 10:1). LC-MS: m/z = 326,4 [M+H]⁺. ¹H NMR (DMSO-d₆, 400 MHz) δ ppm 9.31 (s, 1H), 8.28 (d, J = 8.5 Hz, 2H), 7.74 (d, J = 8.5 Hz, 2H), 7.52 (d, J = 8.5 Hz, 2H), 7.29 (d, J = 8.5 Hz, 2H), 7.03 - 7.05 (d, J = 8.5 Hz, 2H), 6.61 (d, J = 8.5 Hz, 2H), 6.12 (s, 2H). ¹³C NMR (DMSO-d₆, 101 MHz) δ ppm 153.3, 150.3, 148.3, 144.9, 135.0, 129.1, 128.3, 126.4, 117.8, 113.0, 110.9.

4-((4-Bromphenyl)sulfonyl)anilin (**21**): 1-Brom-4-((4-nitrophenyl)sulfonyl)benzol (**18**) (310 mg, 0,91 mmol, 1 Äquiv.) wurde in EtOH/DMSO (4:1 mL) gelöst, Na₂S₂O₄ (937,2 mg, 4,55 mmol, 5 Äquiv.) zugegeben und die Reaktionsmischung für 30 h bei 85 °C gerührt. Nach Abkühlen auf RT wurde die Reaktion unter vermindertem Druck konzentriert, EtOAc und gesättigte NaHCO₃-Lösung zugegeben und der pH-Wert mit NH₄OH auf 9 eingestellt. Die wässrige Phase wurde dreimal mit EtOAc extrahiert und die vereinigten organischen Phasen über MgSO₄ getrocknet. Säulen-chromatographie an Kieselgel (PE/EA) lieferte das gewünschte Produkt als braunen Feststoff (140 mg, 0,44 mmol, 50%). R_{f} = 0,87 (PE/EA, 1:1). ¹H NMR (MeOH-d4, 400 MHz): δ ppm 5.95 - 5.99 (m, 2H), 5.84 - 5.92 (m, 4H), 5.67 (br. s, 2H), 4.97 - 5.02 (m, 2H). ¹³C NMR (MeOH-d4, 101 MHz): δ ppm 206.2, 123.2, 120.5, 119.3, 105.6, 68.1.

N-(4-((4-Bromphenyl)sulfonyl)phenyl)-6-methoxy-2-methylchinolin-4-amin (**22**): 4-Chlor-6-methoxy-2-methylchinolin (7) (100 mg, 0,47 mmol, 1,1 Äquiv.) und 4-((4Bromphenyl)sulfonyl)anilin (**21**) (135 mg, 0,43 mmol, 1 Äquiv.) wurden in absolutem EtOH (6 mL) gelöst, 1 Tropfen konzentrierte HCL zugegeben und entsprechend Methode A umgesetzt, wodurch das Produkt als orangener Feststoff erhalten wurde (190 mg, 0,39 mmol, 89%). R_{f}= 0,26 (PE/EA, 1:1). LC-MS: m/z = 482,95 [M+H]⁺. ¹H NMR (DMSO-d₆, 400 MHz) δ ppm 9.19 (s, 1H), 7.82 - 7.91 (m, 6H), 7.78 (d, J = 9.0 Hz, 1H), 7.52 (d, J = 2.8 Hz, 1H), 7.44 (d, J = 9.0 Hz, 2H), 7.34 (dd, J = 9.0, 2.8 Hz, 1H), 7.21 (s, 1H), 3.87 (s, 3H), 2.49 (s, 3H). ¹³C NMR (DMSO-d₆, 101 MHz) δ ppm 156.2, 147.5, 144.6, 143.9, 141.4, 132.8, 131.3, 130.2, 129.4, 129.0, 127.3, 121.3, 120.4, 118.2, 107.3, 101.1, 59.8, 55.6, 24.7.

6-Methoxy-2-methyl-N-(4-((4-(pyrimidin-4-yl-amin)phenyl)sulfonyl)phenyl) chinolin-4-amin (**24**): N-(4-((4-Bromphenyl)sulfonyl)phenyl)-6-methoxy-2-methylchinolin-4-amin (**22**) (30 mg, 0,062 mmol, 1 Äquiv.), 4-Aminopyrimidin (7 mg, 0,074 mmol, 1,2 Äquiv.), NaOtBu (8,5 mg, 0,087 mmol, 1,4 Äquiv.), Pd₂(dba)₃ (6 mg, 0,006 mmol, 0,1 Äquiv.) und Triphenylphosphan (3,3 mg, 0,018 mmol, 0,3 Äquiv.) wurden entsprechend Methode C in 1,4-Dioxan (2 mL) umgesetzt, wodurch das Produkt als weißer Feststoff erhalten wurde (13,25 mg, 0,027 mmol, 43,5%). R_{f} = 0,37 (DCM/MeOH, 10:1). LC-MS: m/z = 498,2 [M+H]⁺. HR-MS [M+H]⁺ = berechnet 498,15944, gefunden 498,15956 (+0,24720 ppm). ¹H NMR (DMSO-d₆, 500 MHz) δ ppm 10.18 (br. s., 1H), 9.15 (br. s., 1H), 8.71 (s, 1H), 8.29 - 8.39 (m, 2H), 8.02 (d, J = 6.5 Hz, 1H), 7.93 - 7.99 (m, 2H), 7.83 - 7.92 (m, 4H), 7.76 (d, J = 9.2 Hz, 1 H), 7.56 (d, J = 2.3 Hz, 1 H), 7.45 (d, J = 9.2 Hz, 2H), 7.33 (dd, J = 9.2, 2.7 Hz, 1H), 7.18 (s, 1H), 6.92 (d, J = 6.5 Hz, 1H), 6.78 (br. s., 1H), 6.40 (d, J = 7.3 Hz, 1H), 3.87 (s, 3H), 2.48 (s, 3H). ¹³C NMR (DMSO-d₆, 126 MHz) δ ppm 158.1, 157.6, 155.9, 155.6, 154.5, 146.6, 144.4,144.1, 143.9, 134.0, 132.9, 129.9, 128.6, 128.1, 121.0, 120.0, 118.9, 118.2, 108.2, 104.8, 101.0, 55.3, 24.5.

N-(4-((4-((3-(tert-Butyl)-1H-pyrazol-5-yl)amino)phenyl)sulfonyl)phenyl)-6methoxy-2-methylchinolin -4-amin (**25**): N-(4-((4-Bromphenyl)sulfonyl)phenyl)-6-methoxy2-methyl-chinolin-4-amin (**22**) (35 mg, 0,072 mmol, 1 Äquiv.), 3-(tert-Butyl)-1H-pyrazol-5amin (11 mg, 0,081 mmol, 1,2 Äquiv.), NaOtBu (10,5 mg, 0,1 mmol, 1,4 Äquiv.) und PdCl₂(dppf) (6 mg, 0,007 mmol, 0,1 Äquiv.) wurden entsprechend Methode C in 1,4-Dioxan (1,5 mL) umgesetzt, wodurch das Produkt als dunkelbrauner Feststoff erhalten wurde (10 mg, 0,018 mmol, 26%). R_{f}= 0,24 (DCM/MeOH, 10:1). LC-MS: m/z = 542,55 [M+H]⁺. HR-MS [M+H]⁺ = berechnet 542,22204, gefunden 542,22205 (+0,03003 ppm). ¹H NMR (DMSO-d₆, 500 MHz) δ ppm 12.63 (br. s., 1H), 11.92 (br. s., 1H), 9.04 (d, J = 8.8 Hz, 2H), 7.81 (d, J = 8.8 Hz, 2H), 7.76 (d, J = 8.8 Hz, 1H), 7.70 (d, J = 8.8 Hz, 2H), 7.55 (d, J = 2.7 Hz, 1H), 7.39 -7.47 (m, 2H), 7.33 (dd, J = 8.8, 2.7 Hz, 2H), 7.16 (s, H), 3.87 (s, 3H), 1.26 (s, 3H), 1.18 (s, 9H). ¹³C NMR (DMSO-d₆, 126 MHz) δ ppm 155.7, 154.3, 152.6, 131.4, 129.8, 128.3, 128.1, 125.7, 123.9, 120.8, 119.7, 118.2, 113.8, 93.1, 65.9, 55.2, 30.3, 29.7, 29.5, 24.3.

6-Methoxy-N-(4-((4-((4-methoxyphenyl)amin)phenyl)sulfonyl)phenyl)-2-methylchinolin-4-amin (**26**): N-(4-((4-Bromphenyl)sulfonyl)phenyl)-6-methoxy-2-methylchinolin4-amin (**22**) (30 mg, 0,062 mmol, 1 Äquiv.), p-Anisidin (9,15 mg, 0,074 mmol, 1,2 Äquiv.), NaOtBu (8,5 mg, 0,087 mmol, 1,4 Äquiv.), Pd₂(dba)₃ (6 mg, 0,006 mmol, 0,1 Äquiv.) und Triphenylphosphan (3,3 mg, 0,018 mmol, 0,3 Äquiv.) wurden entsprechend Methode C in 1,4-Dioxan (2 mL) umgesetzt, wodurch das Produkt als hellbrauner Feststoff erhalten wurde (24,8 mg, 0,047 mmol, 76%). R_{f}= 0,49 (DCM/MeOH, 10:1). LC-MS: m/z = 526,25 [M+H]⁺. HR-MS [M+H]⁺ = berechnet 526,1795, gefunden 526,17966 (+0,30337 ppm). ¹H NMR (DMSO-d₆, 500 MHz) δ ppm 9.09 (br. s., 1H), 8.62 (s, 1H), 7.81 (d, J = 8.8 Hz, 2H), 7.77 (d, J = 9.2 Hz, 1H), 7.67 (d, J=9.2 Hz, 2H), 7.57 (d, J = 2.3 Hz, 1H), 7.43 (d, J = 8.8 Hz, 2H), 7.34 (dd, J = 9.2, 2.7 Hz, 1H), 7.15 (s, 1H), 7.12 (d, J = 8.8 Hz, 2H), 6.93 (dd, J = 9.2, 2.7 Hz, 4H), 3.88 (s, 3H), 3.74 (s, 3H), 2.49 (s, 3H). ¹³C NMR (DMSO-d₆, 126 MHz): δ ppm 156.0, 150.2, 146.3, 134.1, 129.2, 128.9, 128.6, 123.5, 121.4, 120.2, 118.9, 114.8, 113.1, 101.3, 66.5, 55.7, 55.4, 24.7

Methyl-4-((4-((4-((6-methoxy-2-methylchinolin-4-yl)amino)phenyl)sulfonyl)phenyl)-amino)benzoat (**27**): N-(4-((4-Bromphenyl)sulfonyl)phenyl)-6-methoxy-2-methylchinolin-4-amin (**22**) (30 mg, 0,062 mmol, 1 Äquiv.), Methyl-4-aminobenzoat (11 mg, 0,074 mmol, 1,2 Äquiv.), NaOtBu (8,5 mg, 0,087 mmol, 1,4 Äquiv.), Pd₂(dba)₃ (6 mg, 0,006 mmol, 0,1 Äquiv.) und Triphenylphosphan (3,3 mg, 0,018 mmol, 0,3 Äquiv.) wurden entsprechend Methode C in 1,4-Dioxan (2 mL) umgesetzt, wodurch das Produkt als goldgelber Feststoff erhalten wurde (21,5 mg, 0,039 mmol, 64%). R_{f} = 0,45 (DCM/MeOH, 10:1). LC-MS: m/z = 554,4 [M+H]⁺. HR-MS [M+H]⁺ = berechnet 554,17442, gefunden 554,17486 (+0,79483 ppm). ¹H NMR (DMSO-d₆, 500 MHz) δ ppm 9.33 (s, 1 H), 9.13 (br. s., 1 H), 7.87 (dd, J = 9.2, 8.8 Hz, 4 H), 7.82 (d, J = 9.2 Hz, 2 H), 7.77 (d, J = 9.2 Hz, 1 H), 7.56 (d, J = 2.7 Hz, 1 H), 7.45 (d, J = 8.8 Hz, 2 H), 7.35 (dd, J = 9.2, 2.7 Hz, 1 H), 7.29 (d, J = 8.8 Hz, 2 H), 7.23 (d, J = 8.8 Hz, 2 H), 7.17 (s, 1 H), 3.88 (s, 3 H), 3.81 (s, 3 H), 2.49 (s., 3 H). ¹³C NMR (DMSO-d₆, 126 MHz δ ppm 165.8, 156.2, 146.4, 146.0, 132.4, 130.9, 128.9, 128.7, 121.6, 121.2, 118.6, 116.7, 116.7, 101.2, 66.3, 59.7, 55.5,51.6,24.5,20.7.

6-Brom-2-methylchinolin-4(1H)-on (**31**): Zu einer Suspension von 4-Bromanilin (6,88 g, 40 mmol, 1 Äquiv.) und Ethylacetoacetat (6,63 mL, 52 mmol, 1,3 Äquiv.) in Toluol (75 mL) wurde konzentrierte AcOH (3 mL) zugeben. Unter azeotroper Entfernung von Wasser (Dean-Stark Apparatur) wurde die Reaktionsmischung 2 h unter Rückfluss erhitzt. Anschließend wurde das Lösungsmittel unter vermindertem Druck entfernt und der erhaltene Feststoff in Ph₂O (15 mL) suspendiert und für 1,5 h auf 240 °C erhitzt. Die Reaktionsmischung wurde auf 50 °C abgekühlt und warmes Hexan (50 mL) zugeben. Der entstandene Niederschlag wurde abfiltriert und mit viel Hexan und Aceton gewaschen, wodurch das Produkt als weißer Feststoff (2,26 g, 11,2 mmol, 28%) erhalten wurde. LC-MS: m/z = 238,4 [M+H]⁺. ¹H NMR (DMSO-d₆, 500 MHz) δ ppm 11.69 (s, 1H), 8.10 (d, J = 2.3 Hz, 1H), 7.74 (dd, J = 8.8, 2.3 Hz, 1H), 7.46 (d, J = 8.8 Hz, 1H), 5.95 (s, 1H), 2.33 (s, 3H). ¹³C NMR (DMSO-d₆, 126 MHz) δ ppm 175.29, 150.18, 138.92, 134.08, 126.92, 125.91, 120.33, 115.24, 108.65, 19.43.

6-Brom-4-chlor-2-methylchinolin (**32**): 6-Brom-2-methylchinolin-4(1H)-on (**31**) (1,38 g, 5,46 mmol, 1 Äquiv.) wurde bei 0 °C portionsweise zu einer Überschusslösung von POCl₃ (8 mL) geben. Anschließend wurde die Suspension 30 Min bei RT gerührt und danach 1,5 h unter Rückfluss erhitzt. Der Überschuss an POCl₃ wurde durch Destillation entfernt und der verbliebene Rückstand langsam auf eine Mischung aus Eis und gesättigter NaHCO₃Lösung gegossen und ausgiebig gerührt. Der pH-Wert wurde mit NH₄OH auf 9 eingestellt und der entstandene Niederschlag abgefiltert, wodurch das Produkt als rötliche Feststoff erhalten wurde (1,2 g, 4,69 mmol, 85%). R_{f} = 0,62 (PE/EA, 2:1). LC-MS: m/z = 256,25 [M+H]⁺. ¹H NMR (CDCl₃, 400 MHz) δ ppm 8.31 (d, J = 2.0 Hz, 1H), 7.91 (d, J = 8.9 Hz, 1H), 7.79 (dd, J = 8.9, 2.0 Hz, 1H), 7.40 (s, 1H), 2.71 (s, 3H). ¹³C NMR (CDCl₃, 101 MHz) δ ppm 159.40, 146.93, 141.84, 134.17, 130.53, 126.39, 122.85, 121.09, 25.13.

N-(4-((4-Aminophenyl)sulfonyl)phenyl)acetamid (**34**): Zu einer Lösung von 4,4'Sulfonyldianilin (283 mg, 1,1 mmol, 3 Äquiv.) in Pyridin (1 mL) wurde Ac₂O (36 µL, 0,38 mmol, 1 Äquiv.) und katalytische Mengen DMAP gegeben und die Reaktion für 10 h bei RT gerührt. Säulenchromatographie an Kieselgel (DCM/MeOH) lieferte das gewünschte Produkt als weißen Feststoff (100 mg, 0,034 mmol, 88%). R_{f} = 0,51 (DCM/MeOH, 10:1). LC-MS: m/z = 291,05 [M+H]⁺. ¹H NMR (DMSO-d₆, 400 MHz) δ ppm 10.31 (s, 1H), 7.70 - 7.77 (m, 2H), 7.50 (d, J = 8.8 Hz, 2H), 6.60 (d, J = 8.8 Hz, 2H), 6.13 (s,2H), 2.06 (s, 3H). ¹³C NMR (DMSO-d₆, 101 MHz) δ ppm 169.0, 153.4, 142.9, 136.8, 129.2, 127.7, 126.1, 118.8, 112.9, 24.1, 22.8.

N-(4-((4-((6-Brom-2-methylchinolin-4-yl)amino)phenyl)sulfonyl)phenyl)acetamid (35): 4-Chlor-6-methoxy-2-methylchinolin (7) (95,7 mg, 0,37 mmol, 1,1 Äquiv.) und N-(4((4-Aminophenyl)sulfonyl)phenyl)acetamid (**34**) (10 mg, 0,34 mmol, 1 Äquiv.) wurden in absolutem EtOH (5 mL) gelöst, ein Tropfen konzentrierte HCl zugegeben und entsprechend Methode A umgesetzt, wodurch das Produkt als grüner Feststoff erhalten wurde (65 mg, 0,12 mmol, 37%). R_{f} = 0,45 (DCM/MeOH, 10:1). ¹H NMR (DMSO-d₆, 400 MHz) δ ppm 10.38 (s, 1H), 9.34 (s, 1H), 8.48 (s, 1H), 7.82 - 7.92 (m, 4H), 7.73 - 7.83 (m, 4H), 7.48 (d, J = 8.8 Hz, 1H), 7.24 (s, 5 H), 5.76 (s, 1H), 2,78 (s, 3H) 2.07 (s, 3H). ¹³C NMR (DMSO-d₆, 101 MHz) δ ppm 168.9, 135.1,132.3, 130.6,128.7, 128.2,124.3, 118.8, 54.7, 24.0, 22.1.

N-(4-((4-((2-Methyl-6-(piperidin-1-yl)chinolin-4-yl)amino)phenyl)sulfonyl) phenyl)acetamid (**36**): (a) N-(4-((4-((6-Brom-2-methylchinolin-4-yl)amino)phenyl)-sulfonyl) phenyl)acetamid (**35**) (60 mg, 0,12 mmol, 1 Äquiv.), Piperidin (14 µL, 0,14 mmol, 1,2 Äquiv.), NaOtBu (16 mg, 0,16 mmol, 1,4 Äquiv.) und PdCl₂(dppf) (8 mg, 0,01 mmol, 0,1 Äquiv.) wurden entsprechend Methode C in 1,4-Dioxan (2,5 mL) umgesetzt, wodurch das Produkt als gelber Feststoff erhalten wurde (5 mg, 0,095 mmol, 7,5%). (b) Zu einer Lösung von N-(4-((4-Aminophenyl) sulfonyl)phenyl)-2-methyl-6-(piperidin-1-yl)chinolin-4-amin (**38**) (12 mg, 0,025 mmol, 1 Äquiv.) in Pyridin (0,5 mL) wurde Ac₂O (24 µL, 0,126 mmol, 10 Äquiv.) und katalytische Mengen DMAP gegeben und für 22 h bei RT gerührt. Anschließende Säulenchromatographie an Kieselgel (DCM/MeOH) lieferte das gewünschte Produkt als gelber Feststoff (10 mg, 0,019 mmol, 76%). R_{f}= 0,4 (DCM/MeOH, 10:1). LC-MS: m/z = 515,3 [M+H]⁺. HR-MS [M+H]⁺ = berechnet 515,21114, gefunden 515,21109 (-0,09038 ppm). ¹H NMR (DMSO-d₆, 500 MHz) δ ppm 10.41 (s, 1H), 10.40 (br. s., 1H), 7.81 - 7.89 (m, 4H), 7.77 - 7.81 (m, 2H), 7.69 (d, J = 9.2 Hz, 1H), 7.51 (dd, J = 9.4, 2.5 Hz, 1H), 7.40 (d, J = 8.4 Hz, 1 H), 7.31 - 7.36 (m, 1H), 7.09 (s, 1 H), 3.20 - 3.26 (m, 2H), 2.08 (s, 3H), 1.91 (s, 3H), 1.49 - 1.68 ppm (m, 4H). ¹³C NMR (DMSO-d₆, 126 MHz) δ ppm 172.1, 161.0, 153.8, 150.9, 148.1, 143.7, 140.7, 131.9, 129.0, 128.4, 120.7, 119.1, 113.5, 109.8, 106.3, 101.2, 56.9, 25.4, 24.3, 21.2.

6-Brom-2-methyl-N-(4-((4-(pyridin-4-ylamin)phenyl)sulfonyl)phenyl)chinolin-4amin (37): 6-Brom-4-chlor-2-methylchinolin (**32**) (17,4 mg, 0,067 mmol, 1,1 Äquiv.) und N(4-((4-Aminophenyl)sulfonyl)phenyl)pyridin-4-amin (**20**) (20 mg, 0,062 mmol, 1 Äquiv.) wurden in absolutem EtOH (2 mL) gelöst, ein Tropfen konzentrierte HCl zugegeben und entsprechend Methode A umgesetzt, wodurch das Produkt als gelber Feststoff erhalten wurde (30,5 mg, 0,055 mmol, 87%). R_{f} = 0,18 (DCM/ MeOH, 10:1). LC-MS: m/z = 547,2 [M+H]⁺. ¹H NMR (DMSO-d₆, 400 MHz) δ ppm 9.39 (s, 1 H), 8.49 (d, J = 1.8 Hz, 1H), 8.31 (br. s., 2H), 7.87 (t, J = 8.5 Hz, 2H), 7.75 - 7.81 (m, 2H), 7.49 (d, J = 9.0 Hz, 2H), 7.35 (d, J = 9.0 Hz, 2H), 7.24 (br. s., 1H), 7.07 (d, J = 5.3 Hz, 2H), 2.50 (s, 3H). ¹³C NMR (DMSO-d₆, 101 MHz) δ ppm 150.2, 147.9, 145.4, 133.8, 133.0, 132.3, 130.6, 128.8, 128.6, 124.3, 119.0, 117.5, 111.0, 105.9, 24.8.

N-(4-((4-Aminophenyl)sulfonyl)phenyl)-2-methyl-6-(piperidin-1-yl)chinolin-4amin (**38**): 6-Brom-4-chlor-2-methylchinolin (**32**) (100 mg, 0,39 mmol, 1 Äquiv.), Piperidin (38,7 µL, 0,39 mmol, 1 Äquiv.), Cs₂CO₃ (317 mg, 0,98 mmol, 2,5 Äquiv.), Pd₂(dba)₃ (40 mg, 0,04 mmol, 0,1 Äquiv.) und Triphenylphosphan (21 mg, 0,12 mmol, 0,3 Äquiv.) wurden entsprechend Methode C in 1,4-Dioxan (3 mL) umgesetzt, wobei nach Abfiltern des Katalysators über Celite^{©} das Rohprodukt unter vermindertem Druck konzentriert und anschließend in 3 mL absolutem EtOH gelöst wurde, worauf 4,4'-Sulfonyldianilin (290 mg, 1,17 mmol, 3 Äquiv.) und ein Tropfen konzentrierte HCl zugegeben und die Reaktion entsprechend Methode A weiter umgesetzt wurde. Das Produkt wurde als gelber Feststoff erhalten (18 mg, 0,038 mmol, 10%). R_{f} = 0,35 (DCM/MeOH, 10:1). LC-MS: m/z = 473,15 [M+H]⁺. ¹H NMR (DMSO-d₆, 500 MHz) δ ppm 9.33 (s, 1H), 7.74 - 7.83 (m, 4H), 7.69 (d, J = 8.8 Hz, 2H), 7.54 (d, J = 8.8 Hz, 4H), 7.37 - 7.43 (m, 4H), 7.05 (s, 1H), 6.60 - 6.67 (m, 4H), 6.12 (s, 2H), 5.75 (s, 1H), 3.22 - 3.28 (m, 2H), 2.47 (s, 3H), 1.49 - 1.70 (m, 6H). ¹³C NMR (DMSO-d₆, 126 MHz) δ ppm 159.9, 149.0, 147.9, 144.0, 139.1, 129.4, 128.5, 126.7, 125.5, 124.6, 122.6,119.7, 117.0, 113.3, 50.0, 25.5, 24.2,19.9.

N-(4-((4-Aminophenyl)sulfonyl)phenyl)-2-methyl-6-morpholinchinolin-4-amin (**39**): 6-Brom-4-chlor-2-methylchinolin (**32**) (100 mg, 0,39 mmol, 1 Äquiv.), Morpholin (34 µL, 0,39 mmol, 1 Äquiv.), Cs₂CO₃ (317 mg, 0,98 mmol, 2,5 Äquiv.), Pd₂(dba)₃ (40 mg, 0,04 mmol, 0,1 Äquiv.) und Triphenylphosphan (21 mg, 0,12 mmol, 0,3 Äquiv.) wurden entsprechend Methode C in 1,4-Dioxan (3 mL) umgesetzt, wobei nach Abfiltern des Katalysators über Celite^{©} das Rohprodukt unter vermindertem Druck konzentriert und anschließend in absolutem EtOH (3 mL) gelöst wurde, worauf 4,4'-Sulfonyldianilin (290 mg, 1,17 mmol, 3 Äquiv.) und ein Tropfen konzentrierte HCl zugegeben und die Reaktion entsprechend Methode A weiter umgesetzt wurde. Das Produkt wurde als gelber Feststoff erhalten (46 mg, 0,1 mmol, 25%). R_{f} = 0,3 (DCM/ MeOH, 10:1). LC-MS: m/z = 475,35 [M+H]⁺. ¹H NMR (DMSO-d₆, 400 MHz) δ ppm 9.21 (br. s., 1H), 7.80 (d, J = 8.0 Hz, 2H), 7.74 (d, J = 9.5 Hz, 1H), 7.56 (d, J = 8.0 Hz, 4H), 7.44 (d, J = 7.0 Hz, 2H), 7.09 (s, 1H), 6.64 (d, J = 8.0 Hz, 2H), 6.16 (br. s, 2H), 3.80 (br. s., 4H), 3.25 (br. s., 4H), 2.48 (br. s., 3H). ¹³C NMR (DMSO-d₆, 101 MHz) δ ppm 160.4, 155.4, 153.2, 147.9, 139.8, 128.5, 126.1, 121.4, 119.6, 112.8, 103.4, 82.4, 65.8, 48.5, 25.0.

N-(4-((4-((2-Methyl-6-morpholinchinolin-4-yl)amino)phenyl)sulfonyl)phenyl)acetamid (**40**): Zu einer Lösung von N-(4-((4-Aminophenyl)sulfonyl)phenyl)-2-methyl-6morpholinchinolin-4-amin (**39**) (20 mg, 0,042 mmol, 1 Äquiv.) in Pyridin (0,75 mL) wurde Ac₂O (40 µL, 0,42 mmol, 10 Äquiv.) und katalytische Mengen DMAP gegeben und die Reaktion für 30 h bei RT gerührt. Säulenchromatographie an Kieselgel (DCM/MeOH) lieferte das gewünschte Produkt als gelben Feststoff (15,5 mg, 0,03 mmol, 71%). R_{f} = 0,43 (DCM/MeOH, 10:1). LC-MS: m/z = 517,4 [M+H]⁺. HR-MS [M+H]⁺ = berechnet 517,1904, gefunden 517,19055 (+0,28134 ppm). ¹H NMR (DMSO-d₆, 400 MHz) δ ppm 10.44 (s, 1H), 7.77 - 7.91 (m, 6H), 7.73 (d, J = 8.8 Hz, 1H), 7.55 (d, J = 9.3 Hz, 1H), 7.44 (d, J = 9.3 Hz, 2H), 7.40 (d, J = 3.0 Hz, 5H), 7.24 (d, J = 3.0 Hz, 3H), 7.12 (s, 1H), 3.78 (br. s., 4H), 3.23 (br. s., 4H), 2.47 (s, 3H), 2.08 (s., 3H). ¹³C NMR (DMSO-d₆, 101 MHz) δ ppm 168.9, 147.8, 143.3, 136.4, 135.1, 128.8, 128.7, 128.6, 128.5, 118.7, 75.0, 65.8, 48.5, 23.9.

(4-Bromphenyl)(4-nitrophenyl)methanon (**42**): 4-Nitrobenzoesäurechlorid (2,5 g, 13,5 mmol, 1 Äquiv.) und AlCl₃ (2,69 g, 20,2 mmol, 1,5 Äquiv.) wurden in Brombenzol (3,53 mL, 33,7 mmol, 2,5 Äquiv.) gelöst und für 30 Min bei RT gerührt. Anschließend wurde die Reaktionsmischung über 1,5 h auf 100 °C erhitzt und für weitere 2 h gerührt. Nach Abkühlen auf RT wurde die Reaktion auf Eiswasser gefällt, der entstehende Feststoff abfiltriert und mit viel Wasser gewaschen, wodurch das Produkt als hellbrauner Feststoff erhalten wurde (3,43 g, 11,21 mmol, 83%). R_{f} = 0,81 (PE/EA, 1:1). ¹H NMR (CDCl₃, 400 MHz) δ ppm 7.93 - 7.96 (m, 4H), 7.49 - 7.52 (m, 4H). ¹³C NMR (CDCl₃, 101 MHz): δ ppm 193.4, 149.6, 142.0, 134.6, 131.7, 131.2, 130.3, 128.5, 123.3.

(4-Aminophenyl)(4-bromphenyl)methanon (**43**): Zu einer Lösung von (4Bromphenyl)(4-nitrophenyl)methanon (**42**) (500 mg, 1,64 mmol, 1 Äquiv.) in EtOH/DMSO (6:1,5 mL) wurde Na₂S₂O₄ (1,67g, 8,2 mmol, 5 Äquiv.) zugeben und die Reaktion für 30 h bei 85 °C gerührt. Die Reaktionsmischung wurde unter vermindertem Druck konzentriert, EtOAc und gesättigte NaHCO₃-Lösung zugegeben und der pH-Wert mit NH₄OH auf 9 eingestellt. Die wässrige Phase wurde dreimal mit EtOAc extrahiert und die vereinigten organischen Phasen über MgSO₄ getrocknet. Säulenchromatographie an Kieselgel (DCM/MeOH) lieferte das gewünschte Produkt als weißen Feststoff (75 mg, 0,27 mmol, 17%). R_{f} = 0,7 (PE/EA, 1:1). LC-MS: m/z = 275,1 [M+H]⁺. ¹H NMR (DMSO-d₆, 400 MHz) δ ppm 7.69 - 7.72 (m, 2H), 7.50 - 7.55 (m, 4H), 6.59 - 6.62 (m, 2H), 6.23 (s, 2H). ¹³C NMR (DMSO-d₆, 101 MHz) δ ppm 192.3, 154.0, 138.1, 132.6, 131.2, 130.8, 124.7, 123.2, 112.6.

(4-Bromphenyl)(4-((6-methoxy-2-methylchinolin-4-yl)amino)phenyl)methanon (**44**): 4-Chlor-6-methoxy-2-methylchinolin (7) (61 mg, 0,29 mmol, 1,1 Äquiv.) und (4Aminophenyl)(4-bromphenyl)methanon (**43**) (65 mg, 0,27 mmol, 1 Äquiv.) wurden in absolutem EtOH (3 mL) gelöst, ein Tropfen konzentrierte HCl zugegeben und entsprechend Methode A umgesetzt, wodurch das Produkt als goldgelber Feststoff erhalten wurde (95 mg, 0,212 mmol, 76%). R_{f} = 0,6 (DCM/MeOH, 10:1). LC-MS: m/z = 447,05 [M+H]⁺. ¹H NMR (DMSO-d₆, 500 MHz) δ ppm 9.14 (br. s., 1H), 7.95 (s, 1H), 7.75 - 7.81 (m, 4H), 7.67 (d, J = 8.8 Hz, 2H), 7.62 (s, 1H), 7.44 (d, J = 8.8 Hz, 2H), 7.35 (dd, J = 8.8, 2.9 Hz, 1H), 7.22 (s, 1H), 3.91 (s, 3H), 2.51 (s, 3H). ¹³C NMR (DMSO-d₆, 126 MHz) δ ppm 192.88, 162.0, 156.0, 136.8, 131.7, 131.3, 131.0, 128.9, 125.5, 121.0, 117.9, 101.0, 55.4, 35.5, 30.5.

(4-((6-Methoxy-2-methylchinolin-4-yl)amin)phenyl)(4-(pyridin-4-yl-amino)phenyl)methanon (**45**): (4-Bromphenyl)(4-((6-methoxy-2-methylchinolin-4-yl)amino)phenyl)-methanon (**44**) (20 mg, 0,045 mmol, 1 Äquiv.), 4-Aminopyridin (5,5 mg, 0,053 mmol, 1,2 Äquiv.), NaOtBu (6 mg, 0,064 mmol, 1,4 Äquiv.), Pd₂(dba)₃ (4,5 mg, 0,0045 mmol, 0,1 Äquiv.) und Triphenylphosphan (2,3 mg, 0,013 mmol, 0,3 Äquiv.) wurden entsprechend Methode C in 1,4-Dioxan (1,5 mL) umgesetzt, wodurch das Produkt als gelber Feststoff erhalten wurde (16 mg, 0,035 mmol, 78%). R_{f} = 0,57 (DCM/MeOH, 10:1). LC-MS: m/z = 231,3 [M+H]²⁺. HR-MS [M+H]⁺ = berechnet 461,1972, gefunden 461,19782 (+1,33252 ppm). ¹H NMR (DMSO-d₆, 400 MHz) δ ppm 9.40 (s, 1H), 8.31 (d, J = 5.3 Hz, 2H), 7.74 - 7.85 (m, 5H), 7.69 (d, J = 2.5 Hz, 1H), 7.48 (d, J = 8.8 Hz, 2H), 7.31 - 7.40 (m, 3H), 7.19 (s, 1H), 7.07 - 7.14 (m, 2H), 3.92 (s, 3H), 2.45 ppm (s, 3H). ¹³C NMR (DMSO-d₆, 101 MHz) δ ppm 156.0, 155.6, 149.9, 148.6, 144.7, 131.4, 131.3, 130.7, 130.2, 121.3, 119.6, 118.7, 117.2, 110.6, 105.0, 101.1, 55.5, 24.2.

(4-((6-Methoxy-2-methylchinolin-4-yl)amino)phenyl)(4-(pyrimidin-4-yl-amino) phenyl)methanon (**46**): (4-Bromphenyl)(4-((6-methoxy-2-methylchinolin-4-yl)amino)phenyl)metha-non (**44**) (30 mg, 0,067 mmol, 1 Äquiv.), 4-Aminopyrimidin (8 mg, 0,081 mmol, 1,2 Äquiv.), NaOtBu (9 mg, 0,094 mmol, 1,4 Äquiv.), Pd₂(dba)₃ (6 mg, 0,006 mmol, 0,1 Äquiv.) und Triphenylphosphan (3,5 mg, 0,018 mmol, 0,3 Äquiv.) wurden entsprechend Methode C in 1,4-Dioxan (1,5 mL) umgesetzt, wodurch das Produkt als gelber Feststoff erhalten wurde (21 mg, 0,046 mmol, 69%). R_{f} = 0,45 (DCM/MeOH, 10:1). LC-MS: m/z = 462,35 [M+H]⁺. HR-MS [M+H]⁺ = berechnet 462,19245, gefunden 462,1928 (+0,75802 ppm). ¹H NMR (DMSO-d₆, 500 MHz) δ ppm 10.13 (s, 1H), 8.73 (s, 1H), 8.37 (d, J = 5.7 Hz, 1H), 8.32 (s, 1H), 7.94 (d, J = 8.8 Hz, 2H), 7.76 - 7.83 (m, 4H), 7.70 (d, J = 2.3 Hz, 1H), 7.48 (d, J = 8.8 Hz, 2H), 7.37 (dd, J = 9.2, 2.7 Hz, 1H), 7.19 (s, 1H), 6.95 (dd, J = 5.7, 2.7 Hz, 1 H), 5.75 (s, 1H), 3.93 (s, 3H), 2.52 (s, 3H). ¹³C NMR (DMSO-d₆, 126 MHz) δ ppm 192.7, 174.2, 163.1, 159.4, 158.1, 157.7, 156.1, 155.5, 154.5, 143.5, 131.3, 130.7, 121.3, 118.2, 109.6, 54.7, 54.4, 24.2.

(4-Bromphenyl)(4-nitrophenyl)methanon (**c**)**:** 4-Nitrobenzoesäurechlorid (10 g, 53,9 mmol, 1 Äquiv.) und AlCl₃ (10,8 g, 80,8 mmol, 1,5 Äquiv.) wurden in Brombenzol (14,1 mL, 134 mmol, 2,5 Äquiv.) gelöst und für 30 Min bei RT gerührt. Anschließend wurde die Reaktionsmischung über 1,5 h auf 100 °C erhitzt und für weitere 2 h gerührt. Nach Abkühlen auf RT wurde die Reaktion auf Eiswasser gefällt, der entstehende Feststoff abfiltriert und mit viel Wasser gewaschen, wodurch das Produkt als hellbrauner Feststoff erhalten wurde (14,2 g, 46,4 mmol, 86%). ¹H NMR (CHLOROFORM-d, 500MHz): δ = 7.94 (d, J=8.8 Hz, 4 H), 7.51 ppm (d, J=8.8 Hz, 4 H). ¹³C NMR (CHLOROFORM-d, 101MHz): δ = 193.4, 149.6, 142.0, 134.6, 131.7, 131.2, 130.3, 128.5, 123.3 ppm.

1-Brom-4-(4-nitrobenzyl)benzol (**d**): Zu einer Lösung von (4-Bromphenyl)(4-nitrophenyl)methanon (1g, 3,27 mmol, 1 Äquiv.) in DCM (10 mL) wurde unter Rühren eine Lösung von Trifluormethansulfonsäure (540µL, 6,11 mmol, 1,8 Äquiv.) in DCM (4 mL) zu getropft worauf die tropfenweise Zugabe einer Lösung von Triethylsilan (740, 4,63 mmol, 1,4 Äquiv) in DCM (4 mL) folgte. Die Reaktion wurde 6 h bei RT gerührt und anschließend bei 0 °C auf eine gesättigte Na₂CO₃-Lösung gegossen, dreimal mit DCM extrahiert und die vereinigten organischen Phasen über MgSO₄ getrocknet und das Lösungsmittel unter vermindertem Druck entfernt. Säulenchromatographie an Kieselgel (Petrolether/EtOAc) lieferte das Produkt als weißen Feststoff (690 mg, 2,36 mmol, 72%). GC-MS: 290/292. ¹H NMR (CHLOROFORM-d, 300MHz): δ = 8.11 - 8.20 (m, 2 H), 7.41 - 7.51 (m, 2 H), 7.28 - 7.35 (m, 2 H), 7.05 (d, J=8.8 Hz, 2 H), 4.04 ppm (s, 2 H). ¹³C NMR (CHLOROFORM-d ,75MHz): δ = 148.5, 138.5, 132.3, 131.1, 130.0, 124.3, 121.1, 41.5 ppm.

4-(4-Brombenzyl)anilin (e): Zu einer Lösung von 1-Brom-4-(4-nitrobenzyl)benzol (650 mg, 2,23 mmol, 1 Äquiv.) in EtOH/DMSO (10:2,5 mL) wurde Na₂S₂O₄ (194 g, 11,1 mmol, 5 Äquiv.) zugeben und die Reaktion für 24 h bei 85 °C gerührt. Nach Abkühlen auf RT wurde EtOH aus Reaktionsmischung unter vermindertem Druck entfernt. Anschließend wurde die Reaktion auf Eiswasser gefällt, dreimal mit EtOAc extrahiert und die vereinigten organischen Phasen über MgSO₄ getrocknet und das Lösungsmittel unter vermindertem Druck entfernt. Säulenchromatographie an Kieselgel (Petrolether/EtOAc) lieferte das Produkt als orangener Feststoff (335 mg, 1,28 mmol, 57%). GC-MS: 261/263.

N-(4-(4-Brombenzyl)phenyl)-6-methoxy-2-methylchinolin-4-amin (g): Zu einer Lösung von 4-Chlor-2-methylchinolin (218 mg, 1,05 mmol, 1,1 Äquiv.) und 4-(4-Brombenzyl)anilin (230 mg, 0,88 mmol, 1 Äquiv.) in absolutem EtOH (4 mL) wurde ein Tropfen konzentrierte HCl zugegeben und die Reaktionsmischung für 6 h unter Rückfluss erhitzt. Die Reaktion wurde unter vermindertem Druck konzentriert und der verbliebene Rückstand mit EtOAc und NH₄OH suspendiert und gerührt, bis sämtlicher Feststoff gelöst war. Die wässrige Phase wurde dreimal mit EtOAc extrahiert, die vereinigten organischen Phasen über MgSO₄ getrocknet und das Lösungsmittel unter vermindertem Druck entfernt. Säulenchromatographie an Kieselgel (Petrolether/EtOAc) lieferte das gewünschte Produkt als weißen Feststoff (320 mg, 0,74 mmol, 84%). LC-MS: 433/453. ¹H NMR (DMSO-d₆, 500MHz): δ = 8.60 (s, 1 H), 7.69 (d, J=8.8 Hz, 1 H), 7.66 (d, J=2.7 Hz, 1 H), 7.50 (d, J=8.4 Hz, 2 H), 7.22 - 7.30 (m, 7 H), 6.76 (s, 1 H), 3.93 (s, 2 H), 3.89 (s, 3 H), 2.39 ppm (s, 3 H). ¹³C NMR (DMSO-d₆,75MHz): δ = 156.0, 155.8, 146.9,144.3,141.0,138.9,135.8,131.3,131.0,129.9,129.6,122.5,120.8,119.1,118.7,101.5,101.0, 55.6, 24.9 ppm.

6-Methoxy-2-methyl-N-(4-(4-(pyridin-4-ylamino)benzyl)phenyl)chinolin-4-amin (**63**): In einem ausgeheizten Schlenkkolben mit Rührfisch wurde N-(4-(4-Brombenzyl)phenyl)-6-methoxy-2-methylchinolin-4-amin (20 mg, 0,046 mmol, 1 Äquiv.), Aminopyridin (5,2 mg, 0,06 mmol, 1,2 Äquiv.), KOtBu (7,25 mg, 0,07 mmol) sowie Pd₂dba₃ (4 mg, 6 µmol, 10 mol%) und XPhos (9 mg, 18 µmol, 30 mol%) eingewogen und mit einem Septum verschlossen. Der Kolben wurde dreimal mit Argon evakuiert, entgastes 1,4-Dioxan (2,5 mL) zugegeben und das Septum gegen einen Glasstopfen getauscht. Die Reaktionsmischung wurde danach bei 80 °C für 24 h gerührt. Nach Abkühlen auf RT wurde die Reaktion mit DCM verdünnt, über Celite^{©} gefiltert und mit gesättigter NaHCO₃-Lösung aufgenommen. Die wässrige Phase wurde dreimal mit DCM extrahiert, die vereinigten organischen Phasen über MgSO₄ getrocknet und das Lösungsmittel unter vermindertem Druck entfernt. Säulenchromatographie an Kieselgel (DCM/MeOH) lieferte das Produkt als gelben Feststoff (14 mg, 0,031 mmol, 68%). LC-MS: 224,2/447,3. ¹H NMR (DMSO-d₆, 500MHz): δ = 8.99 (br. s., 1 H), 8.15 (d, J=5.4 Hz, 1 H), 8.03 (d, J=5.7 Hz, 1 H), 7.83 (d, J=2.7 Hz, 1 H), 7.76 (d, J=9.2 Hz, 1 H), 7.29 - 7.37 (m, 4 H), 7.25 (d, J=8.4 Hz, 2 H), 7.16 (d, J=8.4 Hz, 2 H), 6.89 (d, J=6.1 Hz, 1 H), 6.74 (s, 1 H), 6.67 (d, J=6.5 Hz, 1 H), 3.88 - 3.97 (m, 5 H), 2.42 ppm (s, 3 H). ¹³C NMR (DMSO-d₆ ,126MHz): δ = 157.3, 156.0,154.9,150.6,149.0,143.4,138.0,137.9,137.1,135.7,131.6,129.4,122.9,121.4,120.5,118.3, 108.7, 108.6, 101.4, 100.9, 55.7, 23.5 ppm.

6-methoxy-2-methyl-N-(4-(4-(pyrimidin-4-ylamino)benzyl)phenyl)quinolin-4-amine (**68**): In einem ausgeheizten Schlenkkolben mit Rührfisch wurde N-(4-(4-Brombenzyl)phenyl)-6-methoxy-2-methylchinolin-4-amin (60 mg, 0,14 mmol, 1 Äquiv.), Aminopyrimidin (17 mg, 0,18 mmol, 1,2 Äquiv.), KOtBu (22 mg, 0,21 mmol) sowie Pd₂dba₃ (12 mg, 18 µmol, 10 mol%) und XPhos (27 mg, 54 µmol, 30 mol%) eingewogen und mit einem Septum verschlossen. Der Kolben wurde dreimal mit Argon evakuiert, entgastes 1,4 Dioxan (5 mL) zugegeben und das Septum gegen einen Glasstopfen getauscht. Die Reaktionsmischung wurde danach bei 80 °C für 24 h gerührt. Nach Abkühlen auf RT wurde die Reaktion mit DCM verdünnt, über Celite^{©} gefiltert und mit gesättigter NaHCO₃-Lösung aufgenommen. Die wässrige Phase wurde dreimal mit DCM extrahiert, die vereinigten organischen Phasen über MgSO₄ getrocknet und das Lösungsmittel unter vermindertem Druck entfernt. Säulenchromatographie an Kieselgel (DCM/MeOH) lieferte das Produkt als gelben Feststoff (19 mg, 0,044 mmol, 31%). LC-MS: 448.1. ¹H NMR (500 MHz, DMSO) δ = 948; 9.58 (s, 1H), 8.83 (s, 1H), 8.59 (s, 1H), 8.24 (d, J = 5.8 Hz, 1H), 7.79 - 7.67 (m, 2H), 7.61 (d, J = 8.3 Hz, 2H), 7.36 - 7.27 (m, 5H), 7.24 (d, J = 8.3 Hz, 2H), 6.83 - 6.71 (m, 2H), 3.92 (d, J = 10.6 Hz, 5H), 2.42 (s, 3H). ¹³C NMR (126 MHz, DMSO) δ = 948; 160.8, 158.8, 156.9, 156.4, 155.9, 147.3, 139.2, 138.5, 137.9, 136.4, 130.4, 129.8, 123.7, 122.1, 121.1, 119.4, 109.9, 107.8, 102.1, 70.6, 56.6, 25.1.

### Untersuchungen zur Wirksamkeit der erfindungsgemäßen Verbindungen und der Vergleichsverbindung

### Inhibitorische Aktivität gegenüber MtTrxR:

Eine biochemische Evaluierung der inhibitorischen Aktivität (d.h. der IC₅₀-Wert) der erhaltenen Verbindungen gegenüber *MtTrxR* mittels Aktivitätsassay nach *Lu et al.* (Fehlerbreite durch die Standardabweichung angegeben) wurde vorgenommen. Die Testung der potentiellen Inhibitoren gegen *MtTrxR* wurde entsprechend der Beschreibung von *Lu et al.* durchgeführt. Ferner wurden MIC₅₀-Werte (allgemeine *"minimum inhibitory concentration")* bestimmter Verbindungen mit dem BD BACTEC™ MGIT™ gemessen:

| | | | | | | |
|---|---|---|---|---|---|---|
| 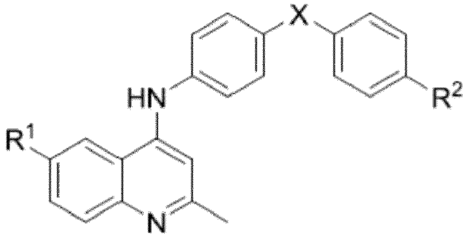 | | | | | | |

| **Verbindung** | **R¹** | **X** | **R²** | **IC₅₀ [µM] DTNB assay** | **MIC₅₀ [µM] (µg/mL)** | **cLogP** |
|---|---|---|---|---|---|---|
| **1** (Vergleich) | MeO | OH | - | 11,54 ± 1,52 | n.b. ** | 3,77 |
| **2** | MeO | SO₂ | 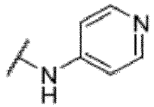 | 0,25 ± 0,04 | 8-32 | 5,18 |
| **8** | MeO | CH₂ | NH₂ | 1,80 ± 0,80 | 4-8 | 5,45 |
| **9** | MeO | SO₂ | NH₂ | 5,21 ± 0,24 | n.b. | 4,25 |
| **10** | MeO | SO₂ | NHAc | 3,59 ± 1,95 | n.b. | 3,96 |
| **24** | MeO | SO₂ | 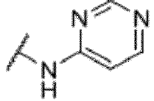 | 0,51 ± 0,04 | 8-32 | 5,30 |
| **25** | MeO | SO₂ | 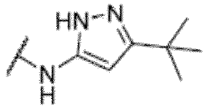 | 9,1 ± 0,30 | n.b. | n.b. |
| **26** | MeO | SO₂ | 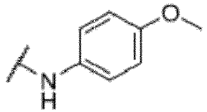 | 1,90 ± 0,80 | 8-32 | 6,39 |
| **27** | MeO | SO₂ | 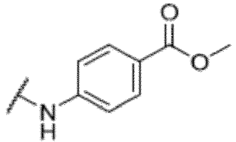 | 4,80 ± 1,60 | n.b. | 6,34 |
| **45** | MeO | CO | 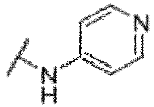 | 1,04 ± 0,20 | >32 | 6,20 |
| **63** | MeO | CH₂ | 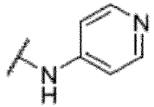 | 5,64 ± 1,73 | 8-32 | 6,38 |
| | | | | 4,70 ± 0,70 | | |
| **68** | MeO | CH₂ | 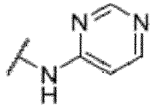 | 2,03 ± 0,65 | 2-8 | 6,50 |
| | | | | 1,70 ± 0,001 | | |

| | | | | | | |
|---|---|---|---|---|---|---|
| * inhibitorische Wirkung ab Konzentrationen von > 10 µM erkennbar ** nicht bestimmt | | | | | | |

Die Daten zeigen, dass mit Verbindung **2** der potenteste Inhibitor identifiziert wurde und zudem durch Acetylierung der Verbindung **9** eine Verbesserung der biologischen Aktivität bewirkt wurde. Eine merkliche Abweichung mit einer besseren Aktivität konnte für Verbindung **8** gefunden werden, für welche ein IC₅₀-Wert von 1,8 µM ermittelt wurde. Diese Bestimmung deckte sich nicht mit der erwarteten Aktivität von 14,7 µM und war bemerkenswert, da davon ausgegangen wurde, dass keine alternative verbrückende Gruppe eine Verbesserung zu einem Sulfon erzielen konnte, was der Vergleich von Verbindung **8** und 10 jedoch widerlegte. Die Testung der CH₂-verbrückten Verbindung **8** lieferte somit einen vielversprechenden Kandidaten für eine weitere Optimierung, da zu erwarten war, dass die Erhöhung der Lipophilie durch die Methylgruppe eine verbesserte Permeabilität für die mykobakterielle Zellwand darstellen sollte. Die sulfonverbrückten Verbindungen **24, 25, 26** und **27** zeigen gegenüber Verbindung **2** zwar einen Verlust an Aktivität, sind jedoch trotzdem potentere Inhibitoren als Vergleichsverbindung **1.** Auch die carbonylverbrückte Verbindung **45** ist ein potenterer Inhibitor als Vergleichsverbindung 1. Ferner sind die CH₂-verbrückten Verbindungen 63 und 68 potentere Inhibitoren als Vergleichsverbindung 1.

### Permeabilität:

Ausgewählte Verbindungen wurden auf ihre Permeabilität mithilfe des PAMPA-Assays (Parallel Artificial Membrane Permeation Assay) untersucht. Die Permeabilität der Verbindungen gegenüber einer artifiziellen Membran aus Phospholipiden wurde in einer Filterplatte getestet. Da ein signifikanter Unterschied zwischen der artifiziellen Membran und der mykobakteriellen Zellwand bestand, sollten die Werte nur als Maß genutzt werden, um die Permeabilität der Verbindungen zueinander abwägen zu können. Dafür wurde als Referenzwert die Verteilung zwischen einer Donor- und Akzeptorkammer ohne Membran bestimmt (100% Flux), wobei die Konzentration der Verbindungen in den Kammern spektralphotometrisch ermittelt wurde. Anschließend wurde die Permeabilität der Verbindungen 1, 8, 63 und 68 gegenüber der eingesetzten Membran ermittelt. Der PAMPA-Flux-[%]-Wert betrug für Verbindung 1 12,60, für Verbindung 8 29,70, für Verbindung 63 53,60 und für Verbindung 68 41,00. Es lässt sich festhalten, dass Verbindungen 8, 63 und 68 eine deutlich höhere Permeabilität als Vergleichsverbindung 1 aufweisen.

### Antimykobakterielle Aktivität:

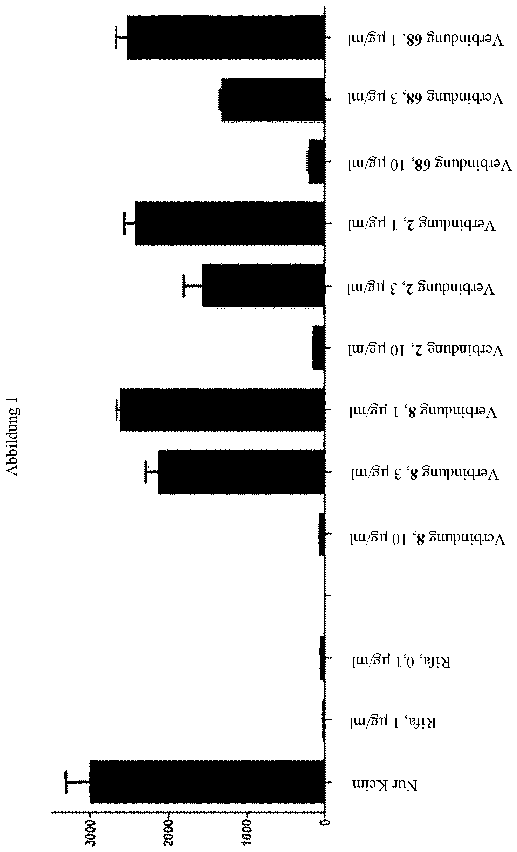

Abbildung 1 zeigt, dass die erfindungsgemäßen Verbindungen 2, 8 und 68 eine konzentrationsabhängige antimykobakterielle Aktivität aufweisen.

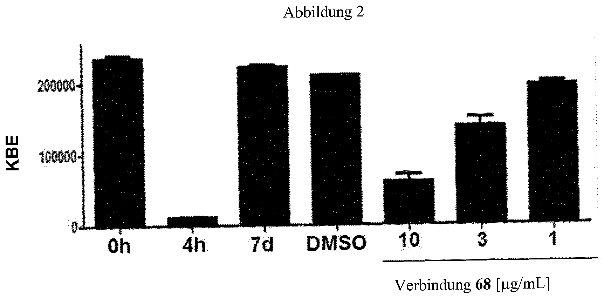

Abbildung 2 zeigt, dass die erfindungsgemäße Verbindung 68 eine konzentrationsabhängige antimykobakterielle Aktivität gegenüber dem *Mycobacterium-tubercolosis-*Strang H37Rv in infizierten humanen Makrophagen aufweist.

### Zytotoxizität:

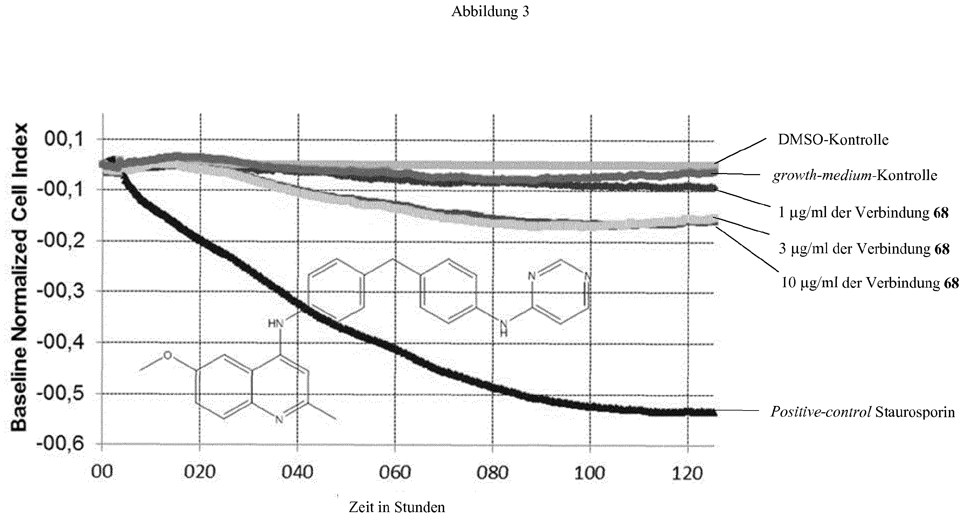

Abbildung 3 zeigt, dass die erfindungsgemäße Verbindung 68 eine konzentrationsabhängige Wachstumshemmung in infizierten Makrophagen aufweist, ohne die Makrophagen zu beeinflussen, d.h. ohne toxisch wirksam zu sein.

## Patentansprüche

1. Eine Verbindung der allgemeinen Formel (1) 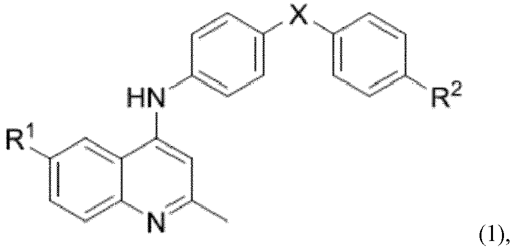 worin
R¹ und R² jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NHC(=O)N(R₀)₂;
X für -CH₂-, -S(=O)₂- oder -C(=O)- steht;
wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf);
wobei in Bezug auf "Aliphat" und "Cycloaliphat" unter "ein- oder mehrfach substituiert" die ein-oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O, -Ro, -C(=O)R₀, -C(=O)H, -C(=O)OH, -C(=O)OR₀, -C(=O)NH₂, -C(=O)NHR₀, -C(=O)N(R₀)₂, -OH, -ORo, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀, -NH-C(=O)N(R₀)₂, -Si(R₀)₃ und -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die ArylSubstituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -Ro, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃ oder -PO(OR₀)₂ verstanden wird; wobei ggf. vorhandene N-Ringatome jeweils oxidiert sein können;
Ro jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
oder ein physiologisch verträgliches Salz davon.

2. Die Verbindung nach Anspruch 1, worin
R¹ und R² jeweils unabhängig voneinander ausgewählt sind aus der Gruppe bestehend aus -H, -F, -Cl, -Br, -I, -R₀, -C(=O)R₀, -C(=O)OR₀, -C(=O)NHR₀, -C(=O)N(R₀)₂, -ORo, -OC(=O)R₀, -OC(=O)OR₀, -OC(=O)NHR₀, -OC(=O)N(R₀)₂, -SRo, -S(=O)₁₋₂-R₀, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NH-C(=O)N(R₀)₂; wobei R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl oder -C₁₋₈-Aliphat-Heteroaryl steht.

3. Die Verbindung nach Anspruch 1 oder 2, worin
X für -CH₂- oder -S(=O)₂- steht;
R¹ ausgewählt ist aus der Gruppe bestehend aus -Ro, -C(=O)R₀, -C(=O)OR₀, -C(=O)NHR₀, -C(=O)N(R₀)₂, -ORo, -OC(=O)R₀, -OC(=O)OR₀, -OC(=O)NHR₀ und -OC(=O)N(R₀)₂; wobei R₀ jeweils unabhängig für -C₁₋₈-Aliphat, -Aryl, -Heteroaryl, -C₁₋₈-Aliphat-Aryl oder -C₁₋₈-Aliphat-Heteroaryl steht; und
R² ausgewählt ist aus der Gruppe bestehend aus -NH₂, -NHR₀, -N(R₀)₂, -N⁺(R₀)₃ und -N⁺(R₀)₂O⁻ ; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -C₃₋₁₂-Cycloaliphat, -Aryl, -Heteroaryl
oder -C₁₋₈-Aliphat-C₃₋₁₂-Cycloaliphat steht;
oder
X für -C(=O)- steht;
R¹ ausgewählt ist aus der Gruppe bestehend aus -R₀, -C(=O)R₀, -C(=O)OR₀, -C(=O)NHR₀, -C(=O)N(R₀)₂, -OR₀, -OC(=O)R₀, -OC(=O)OR₀, -OC(=O)NHR₀ und -OC(=O)N(R₀)₂; wobei R₀ jeweils unabhängig für -C₁₋₈-Aliphat, -Aryl, -Heteroaryl, -C₁₋₈-Aliphat-Aryl oder -C₁₋₈-Aliphat-Heteroaryl steht; und
R² ausgewählt ist aus der Gruppe bestehend aus -NH₂, -NHR₀, -N(R₀)₂, -N⁺(R₀)₃ und -N⁺(R₀)₂O⁻ ; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl, -Oxadiazolyl, -C₃₋₁₂-Cycloaliphat, -Aryl oder -C₁₋₈-Aliphat-C₃₋₁₂-Cycloaliphat steht.

4. Die Verbindung nach einem der Ansprüche 1 bis 3, worin
X für -CH₂- oder -S(=O)₂- steht;
R¹ ausgewählt ist aus der Gruppe bestehend aus -R₀, -ORo, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und
R² ausgewählt ist aus der Gruppe bestehend aus -NH₂, -NHR₀, -N(R₀)₂, -N⁺(R₀)₃ und -N⁺(R₀)₂O⁻ ; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -Aryl oder-Heteroaryl steht;
oder
X für -C(=O)- steht;
R¹ ausgewählt ist aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und
R² ausgewählt ist aus der Gruppe bestehend aus -NH₂, -NHR₀, -N(R₀)₂, -N⁺(R₀)₃ und -N⁺(R₀)₂O⁻ ; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht.

5. Die Verbindung nach einem der Ansprüche 1 bis 4, worin
X für -CH₂- oder -S(=O)₂- steht;
R¹ ausgewählt ist aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und
R² ausgewählt ist aus der Gruppe bestehend aus -NH₂, -NHR₀, -N(R₀)₂, -N⁺(R₀)₃ und -N⁺(R₀)₂O⁻ ; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -Phenyl, -Naphthyl, -Anthracenyl, -Phenanthrenyl, -Fluoranthenyl, -Fluorenyl, -Indanyl, -Tetralinyl, -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrimidinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl
steht;
oder
X für -C(=O)- steht;
R¹ ausgewählt ist aus der Gruppe bestehend aus -R₀, -OR₀, -OC(=O)R₀, -OC(=O)OR₀, -OC(=O)NHR₀ und -OC(=O)N(R₀)₂; wobei Ro jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und
R² ausgewählt ist aus der Gruppe bestehend aus -NH₂, -NHR₀, -N(R₀)₂, -N⁺(R₀)₃ und -N⁺(R₀)₂O⁻ ; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht.

6. Die Verbindung nach einem der Ansprüche 1 bis 5, worin
X für -CH₂- oder -S(=O)₂- steht;
R¹ ausgewählt ist aus der Gruppe bestehend aus -R₀, -OR₀, -OC(=O)R₀, -OC(=O)OR₀, -OC(=O)NHR₀ und -OC(=O)N(R₀)₂; wobei Ro jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und
R² ausgewählt ist aus der Gruppe bestehend aus -NH₂, -NHR₀, -N(R₀)₂, -N⁺(R₀)₃ und -N⁺(R₀)₂O⁻ ; wobei Ro jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃, -CH=CHCH=CH₂, jeweils unsubstituiert, -Phenyl, -Naphthyl, -Anthracenyl, -Phenanthrenyl, -Fluoranthenyl, -Fluorenyl, -Indanyl, -Tetralinyl, -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrimidinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl
steht;
oder
X für -C(=O)- steht;
R¹ ausgewählt ist aus der Gruppe bestehend aus -R₀, -OR₀, -OC(=O)R₀, -OC(=O)OR₀, -OC(=O)NHR₀ und -OC(=O)N(R₀)₂; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht; und
R² ausgewählt ist aus der Gruppe bestehend aus -NH₂, -NHR₀, -N(R₀)₂, -N⁺(R₀)₃ und -N⁺(R₀)₂O⁻ ; wobei R₀ jeweils unabhängig für -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl oder -Oxadiazolyl steht;
und -Phenyl, -Naphthyl, -Anthracenyl, -Phenanthrenyl, -Fluoranthenyl, -Fluorenyl, -Indanyl, -Tetralinyl, -Pyrrolyl, -Indolyl, -Furyl, -Benzofuranyl, -Thienyl, -Benzothienyl, -Benzothiadiazolyl, -Benzooxadiazolyl, -Benzothiazolyl, -Benzooxazolyl, -Benzotriazolyl, -Benzodioxolanyl, -Benzodioxanyl, -Phtalazinyl, -Pyrazolyl, -Imidazolyl, -Thiazolyl, -Oxazolyl, -Isoxazoyl, -Pyridinyl, -Pyridazinyl, -Pyrimidinyl, -Pyrazinyl, -Pyranyl, -Indazolyl, -Purinyl, -Indolizinyl, -Chinolinyl, -Isochinolinyl, -Chinazolinyl, -Carbazolyl, -Phenazinyl, -Phenothiazinyl und -Oxadiazolyl jeweils unabhängig voneinander unsubstituiert oder ein- fach substituiert sind durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀ und -OR₀ verstanden wird, wobei ggf. vorhandene N-Ringatome jeweils oxidiert sein können; wobei R₀ jeweils unabhängig für -CH₃, -C(=O)CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂CH₂CH₃, -CH₂CH₂CH₂CH₂CH₂CH₃, -CH=CH₂, -C≡CH, -CH₂CH=CH₂, -CH=CH-CH₃, -CH₂C≡CH, -C≡CCH₃ und -CH=CHCH=CH₂, jeweils unsubstituiert, steht.

7. Die Verbindung nach einem der Ansprüche 1 bis 6, worin
X für -CH₂- oder -S(=O)₂- steht; oder
X für -CH₂- oder -C(=O)- steht; oder
X für -S(=O)₂- oder -C(=O)- steht; oder
X für -CH₂- steht; oder
X für -S(=O)₂- steht; oder
X für -C(=O)- steht.

8. Die Verbindung nach Anspruch 1 oder 2, ausgewählt aus der Gruppe bestehend aus
6-Methoxy-2-methyl-N-(4-((4-(pyridin-4-ylamino)phenyl)sulfonyl)phenyl) chinolin-4-amin (**2**);
N-(4-(4-Aminobenzyl)phenyl)-6-methoxy-2-methylchinolin-4-amin (**8**);
N-(4-((4-Aminophenyl)sulfonyl)phenyl)-6-methoxy-2-methylchinolin-4-amin (**9**);
N-(4-((4-((6-Methoxy-2-methylchinolin-4-yl)amin)phenyl)sulfonyl)phenyl)acetamid (**10**);
4-Butyl-N-(4-((4-((6-methoxy-2-methylchinolin-4-yl)amin)phenyl)sulfonyl)phenyl)benzamid (**11**);
1-Benzyl-3-(tert-butyl)-N-(4-((4-((6-methoxy-2-methylchinolin-4-yl)amin)phenyl)sulfonyl)-phenyl)-1H-pyrazol-5-carboxamid (**12**);
6-Methoxy-2-methyl-N-(4-((4-(pyrimidin-4-yl-amin)phenyl)sulfonyl)phenyl)chinolin-4-amin (**24**);
N-(4-((4-((3-(tert-Butyl)-1H-pyrazol-5-yl)amino)phenyl)sulfonyl)phenyl)-6methoxy-2-methyl-chinolin-4-amin (**25**);
6-Methoxy-N-(4-((4-((4-methoxyphenyl)amin)phenyl)sulfonyl)phenyl)-2-methylchinolin-4-amin (**26**);
Methyl-4-((4-((4-((6-methoxy-2-methylchinolin-4-yl)amino)phenyl)sulfonyl)phenyl)-amino)-benzoat (**27**);
N-(4-((4-((2-Methyl-6-(piperidin-1-yl)chinolin-4-yl)amino)phenyl)sulfonyl)phenyl)acetamid (**36**);
6-Brom-2-methyl-N-(4-((4-(pyridin-4-ylamin)phenyl)sulfonyl)phenyl)chinolin-4amin (**37**);
N-(4-((4-((2-Methyl-6-morpholinchinolin-4-yl)amino)phenyl)sulfonyl)phenyl)acetamid (40); (4-((6-Methoxy-2-methylchinolin-4-yl)amin)phenyl)(4-(pyridin-4-yl-amino)phenyl)methanon (**45**);
(4-((6-Methoxy-2-methylchinolin-4-yl)amino)phenyl)(4-(pyrimidin-4-yl-amino)phenyl)-methanon (**46**);
6-Methoxy-2-methyl-N-(4-(4-(pyridin-4-ylamino)benzyl)phenyl)chinolin-4-amin (**63**);
6-methoxy-2-methyl-N-(4-(4-(pyrimidin-4-ylamino)benzyl)phenyl)quinolin-4-amine (**68**); und deren physiologisch verträglichen Salzen.

9. Die Verbindung nach einem der Ansprüche 1 bis 8, ausgewählt aus der Gruppe bestehend aus
6-Methoxy-2-methyl-N-(4-((4-(pyridin-4-ylamino)phenyl)sulfonyl)phenyl) chinolin-4-amin (**2**);
N-(4-(4-Aminobenzyl)phenyl)-6-methoxy-2-methylchinolin-4-amin (**8**);
N-(4-((4-Aminophenyl)sulfonyl)phenyl)-6-methoxy-2-methylchinolin-4-amin (**9**);
N-(4-((4-((6-Methoxy-2-methylchinolin-4-yl)amin)phenyl)sulfonyl)phenyl)acetamid (**10**); 6-Methoxy-2-methyl-N-(4-((4-(pyrimidin-4-yl-amin)phenyl)sulfonyl)phenyl)chinolin-4-amin (**24**);
N-(4-((4-((3-(tert-Butyl)-1H-pyrazol-5-yl)amino)phenyl)sulfonyl)phenyl)-6methoxy-2-methyl-chinolin-4-amin (**25**);
6-Methoxy-N-(4-((4-((4-methoxyphenyl)amin)phenyl)sulfonyl)phenyl)-2-methylchinolin-4-amin (**26**);
Methyl-4-((4-((4-((6-methoxy-2-methylchinolin-4-yl)amino)phenyl)sulfonyl)phenyl)-amino)-benzoat (**27**);
(4-((6-Methoxy-2-methylchinolin-4-yl)amin)phenyl)(4-(pyridin-4-yl-amino)phenyl)methanon (**45**);
6-Methoxy-2-methyl-N-(4-(4-(pyridin-4-ylamino)benzyl)phenyl)chinolin-4-amin (**63**);
6-methoxy-2-methyl-N-(4-(4-(pyrimidin-4-ylamino)benzyl)phenyl)quinolin-4-amine (**68**); und deren physiologisch verträglichen Salzen.

10. Die Verbindung nach einem der Ansprüche 1 bis 9, ausgewählt aus der Gruppe bestehend aus
6-Methoxy-2-methyl-N-(4-((4-(pyridin-4-ylamino)phenyl)sulfonyl)phenyl) chinolin-4-amin (**2**);
6-Methoxy-2-methyl-N-(4-((4-(pyrimidin-4-yl-amin)phenyl)sulfonyl)phenyl)chinolin-4-amin (**24**);
N-(4-((4-((3-(tert-Butyl)-1H-pyrazol-5-yl)amino)phenyl)sulfonyl)phenyl)-6methoxy-2-methyl-chinolin-4-amin (**25**);
6-Methoxy-N-(4-((4-((4-methoxyphenyl)amin)phenyl)sulfonyl)phenyl)-2-methylchinolin-4-amin (**26**);
Methyl-4-((4-((4-((6-methoxy-2-methylchinolin-4-yl)amino)phenyl)sulfonyl)phenyl)-amino)-benzoat (**27**); und
deren physiologisch verträglichen Salzen;
und/oder
6-Methoxy-2-methyl-N-(4-(4-(pyridin-4-ylamino)benzyl)phenyl)chinolin-4-amin (**63**); 6-methoxy-2-methyl-N-(4-(4-(pyrimidin-4-ylamino)benzyl)phenyl)quinolin-4-amine (**68**); und deren physiologisch verträglichen Salzen.

11. Arzneimittel enthaltend wenigstens eine Verbindung nach einem der Ansprüche 1 bis 10 oder ein physiologisch verträgliches Salz davon, sowie ggf. geeignete Zusatz- und/oder Hilfsstoffe und/oder ggf. weitere Wirkstoffe.

12. Die Verbindung nach einem der Ansprüche 1 bis 10 und/oder das Arzneimittel nach Anspruch 11 zur Anwendung als Medikament.

13. Die Verbindung nach einem der Ansprüche 1 bis 10 und/oder das Arzneimittel nach Anspruch 11 zur Anwendung bei der Behandlung von Tuberkolose.

14. Die Verbindung nach Anspruch 13 und/oder das Arzneimittel nach Anspruch 13 zur Anwendung bei der Behandlung von Tuberkolose, wobei Tuberkolose ausgewählt ist aus der Gruppe bestehend aus
- Tuberkulose der Atmungsorgane,
- Tuberkulose des Nervensystems,
- Tuberkulose sonstiger Organe, und
- Miliartuberkulose.

15. Die Verbindung nach Anspruch 14 und/oder das Arzneimittel nach Anspruch 14 zur Anwendung bei der Behandlung von Tuberkolose, wobei
- Tuberkulose der Atmungsorgane ausgewählt ist aus der Gruppe bestehend aus
- Lungentuberkulose,
- Tuberkulose der intrathorakalen Lymphknoten,
- Tuberkulose des Larynx, der Trachea und der Bronchien, und
- Tuberkulöser Pleuritis;
und/oder
- Tuberkulose des Nervensystems ausgewählt ist aus der Gruppe bestehend aus
- Tuberkulöser Meningitis, und
- Meningealem Tuberkulom;
und/oder
- Tuberkulose sonstiger Organe ausgewählt ist aus der Gruppe bestehend aus
- Tuberkulose der Knochen und Gelenke,
- Tuberkulose des Urogenitalsystems,
- Tuberkulose peripherer Lymphknoten,
- Tuberkulose des Darmes, des Peritoneums und der Mesenteriallymphknoten,
- Tuberkulose der Haut und des Unterhautgewebes,
- Tuberkulose des Auges,
- Tuberkulose des Ohres, und
- Tuberkulose der Nebennieren.
